# EUROPEAN PATENT APPLICATION

(11) **EP 1 403 269 A1**
(43) Date of publication of application: **31.03.2004**
(21) Application number: 02733468.9
(22) Date of filing: 11.06.2002
(51) Int. Cl.: C07D 413/12, C07D 413/14, A61K 31/536, A61P 31/12, A61P 31/20, A61P 31/22, A61P 43/00

(54) **REMEDIAL AGENT FOR VIRAL INFECTIOUS DISEASE**

(30) Priority: 13.06.2001 JP 2001179282; 12.12.2001 JP 2001379282
(71) Applicant: Asahi Kasei Pharma Corporation, Tokyo 101-8481 (JP)
(72) Inventor: TAKAHASHI, Wataru, Kannami-cho, Tagata-gun, Shizuoka 419-01 (JP); WATANABE, Naoto, Tagata-gun, Shizuoka 410-2121 (JP); SAITO, Yasuyoshi, Mishima-shi, Shizuoka 411-0803 (JP)
(74) Representative: Forstmeyer, Dietmar, Dr. rer. nat., Dipl.-Chem.
(86) International application number: PCT/JP2002/005795
(87) International publication number: WO 2003/002558

(57) **Abstract**

A pyridine ring-containing benzoxazinone derivative or a pharmaceutically acceptable salt or tautomer thereof represented by general formula (1): 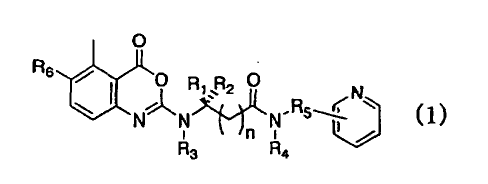 (wherein R₁ to R₆ and n are as defined in the description) has excellent inhibitory activity against a protease. Thus, the benzoxazinone derivative or a pharmaceutically acceptable salt thereof is useful as an active ingredient for a therapeutic agent for virus infections, particularly herpes virus infections.

## Description

### Technical Field

The present invention relates to a pyridine ring-containing benzoxazinone derivative or a pharmaceutically acceptable salt or tautomer thereof and a pharmaceutical composition comprising the same for treating virus infections such as diseases associated with herpes viruses.

### Background Art

A novel method for treating viral diseases has been strongly desired. Methods for treating bacterial infections have developed considerably, although there remains substantially no innovative method for treating virus infections. Zidovudine is the first approved agent for treating diseases associated with human immunodeficiency viruses. At present, Ganciclovir, Aciclovir, vidaravine, Foscarnet, Valacyclovir, penciclovir, docosanol, Famciclovir, and the like are used for treating herpes virus infections. These therapeutic agents, however, sometimes have side-effects caused by DNA replication interference in host cells. Also, the types of viruses upon which these therapeutic agents effectively act are limited. Further, viruses are known to become resistant to medical agents, and this gradually lowers medicinal effects. Thus, it has been desired to develop a method of combination therapy for herpes infections, which has greater therapeutic effects and safety and a lower frequency of the development of viruses resistant thereto than the effects attained by conventional clinical treatments using a single agent.

Viruses are classified into a wide range of categories based on their incorporation of RNA or DNA. Viruses classified as a DNA type include Adenoviridae, Poxviridae, Papoviridae, and Herpesviridae.

Herpesviridae is a genus of DNA virus, and examples thereof include herpes simplex virus type-1 (abbreviated to HSV-1), herpes simplex virus type-2 (abbreviated to HSV-2), cytomegarovirus (abbreviated to CMV), herpes varicella-zoster virus (abbreviated to VZV; herpes zoster virus), Epstein-Barr virus, human herpes virus-6 (HHV-6), human herpes virus-7 (HHV-7), human herpes virus-8 (HHV-8), pseudorabies, rhinotracheitis, and herpes B virus.

It is known that the herpes virus instructs syntheses of many proteins encoded by herpes virus DNA in host cells, thereby expressing the genetic contents thereof. An important virus-encoded protein has a protease site on the N-terminal side and an assembly protein on the C-terminal side. This structure realizes the generation of a protease and an assembly protein that are autocatalytically cleaved and shortened at a site of a specific amino acid sequence known as a "release" site. The assembly protein is further cleaved by a protease at another site of a specific amino acid sequence known as a "mature" cleavage site. In EP No. 514,830 (issued on November 25, 1992), a virus-specific serine protease, which plays a role in herpes virus replication, is disclosed. Further, Lui and Roizman disclose the sequence and activity of a related assembly protein encoded by this protease and UL26 of HSV-1 (J. Virol, 65, 5149 (1991)). A. R. Welch et al. disclose the assembly protein encoded by a related protease (also known as an assembly protein, Proc. Natl. Acad. Sci. USA, 88, 10792 (1991) and WO 93/01291, issued on January 21, 1993) and UL80 of CMV. Under these circumstances, the development of herpes virus protease (assembly protease) inhibitors is currently actively attempted as novel therapeutic agents for herpes virus infections.

Several 4H-3,1-benzoxazinone compounds are reported to have serine protease inhibitory activities. For example, Teshima et al. disclose various 2-alkyl-4H-3,1-benzoxazin-4-one compounds as enzyme inhibitors (J. Biol. Chem., 257, 5085 to 5091 (1982)). Moorman and Abeles disclose 4H-3,1-benzoxazine-2,4-dione compounds as those having several types of enzyme inhibitory activities (J. Amer. Chem. Soc., 104, 6785 to 6786 (1982)). R. Stein et al. disclose 2-alkyl-4H-benzoxazin-4-one compounds further having substituents at positions 5, 6, and 7 as those inhibiting elastase enzymes (Biochemistry, 26, 4126 to 4130 (1987)). In WO 92/18488 (disclosed on October 29, 1992), a 2-alkyl-4H-3,1-benzoxazin-4-one compound having substituents at positions 5 and 7 is disclosed as a selective elastase inhibitor. In European Patent Application No. 206,323 (disclosed on December 30, 1985), 2-alkoxy-, 2-aryloxy-, and 2-aralkoxy-4H-3,1-benzoxazin-4-one compounds having substituents at positions 5, 6, 7, and 8 are disclosed as enzyme inhibitors. A. Kranz et al. disclose 2-alkoxy-, 2-aryloxy-, and 2-aralkoxy-4H-3,1-benzoxazin-4-one compounds further having substituents at positions 5, 7, and 8 as enzyme inhibitors (U.S. Patent No. 4,745,116). C. Hiebert et al. disclose a 6-(amino acid)amino-2-alkoxybenzoxazinone compound as an elastase inhibitor (U.S. Patent No. 5,428,021). In WO 96/07648 (disclosed on March 14, 1996), a 2-phenylamino-benzoxazinone compound for treating the Alzheimer's disease, more specifically a 6-chloro-2-(2-iodophenylamino)-benzo[d][1,3]oxazin-4-one compound, is disclosed.

A 2-amino-4H-3,1-benzoxazinone compound has been already disclosed. A. Kranz et al. disclose a 4H-3,1-benzoxazin-4-one compound, wherein the position 2 thereof is substituted with an alkyl, alkylamino, alkoxy, or alkylthio substituent and substituents are present at positions 5, 6, and 7, as an elastase inhibitor (J. Med. Chem., 33, 464 to 479 (1990)). Uejima et al. disclose a 2-alkylamino-5-methyl-7-acylamino-4H-3,1-benzoxazin-4-one compound as a highly selective elastase inhibitor having high plasma stability (J. Pharm. Exp. Ther., 265, 516 to 522 (1993)). A. Kranz et al. disclose 2-alkylamino- and 2-alkylureido-4H-3,1-benzoxazin-4-one compounds further having substituents at positions 5, 7, and 8 as enzyme inhibitors (U.S. Patent No. 4,657,893).

F. L. M. Alvarez discloses the production of a 2-sulfonylamino-4H-3,1-benzoxazinone compound (An. Quim., 79, 115 to 17 (1983)). J. G. Tercero et al. disclose the production of a 2-arylsulfonylamino-4H-3,1-benzoxazinone compound (An. Quim., 83, 247 to 50 (1987)).

I. Butula et al. disclose the synthesis of a 2-alkylamino-4H-3,1-benzoxazinone compound (Croat. Chem. Acta., 54, 105 to 8 (1981)). H. Ulrich et al. disclose the synthesis of a 2-alkylamino-4H-3,1-benzoxazinone compound (J. Org. Chem., 32, 4052 to 53 (1967)). E. Papadopoulos discloses the use of a 2-haloalkylamino-4H-3,1-benzoxazin-4-one compound as a starting material for synthesizing phenylurea (J. Heterocyclic. Chem., 21, 1411 to 14 (1984)). In European Patent Application No. 466,944 (disclosed on January 22, 1992), a 2-alkylamino-7-acylamino-5-alkyl-4H-benzoxazin-4-one compound is disclosed as a selective enzyme inhibitor for elastase. M. Badawy et al. disclose the use of an N-phenyl-2-amino-4H-3,1-benzoxazin-4-one compound as a starting material for synthesizing a quinazoline compound (J. Heterocyclic. Chem., 21, 1403 to 4 (1984)). R. Khan et al. disclose the synthesis of a 2-[5-aryl-1,3,4-oxadiazol-2-yl]amino-4H-3,1-benzoxazin-4-one compound (J. Chem. Research (S), 342 to 43 (1992)).

Recently, a 4H-3,1-benzoxazin-4-one compound was reported as a herpes virus assembly protease inhibitor. N. Abood et al. disclosed the 4H-3,1-benzoxazin-4-one compound as a selective assembly protease inhibitor (WO 96/37485), and Kasai et al. further disclosed it as an antiviral agent against the herpes simplex virus (WO 01/03697).

Recently, an increasing number of reports have been made on herpes virus assembly protease inhibitors having other skeletons. However, the protease inhibitor that was disclosed by A. D. Borthwick et al. was the only one that had antiherpes virus activity (WO 00/18770). Further, no report has been made that specifically indicates their effectiveness using animal models or their effectiveness as medicine. There is no known example that shows that the use of an assembly protease inhibitor in combination with another antiviral agent enhances therapeutic effects in animal models.

An object of the present invention is to provide a protease inhibitor having antiviral activity in an animal model. It is another object of the present invention to provide an agent for combination therapy that exhibits synergistic inhibitory activities on virus multiplication in cell lines and has enhanced therapeutic effects in animal models when it is used in combination with another antiviral agent.

### Disclosure of the Invention

The present inventors have conducted concentrated studies in order to attain the above objects. As a result, they have found that a 4H-3,1-benzoxazin-4-one derivative having a pyridine ring at position 2 via an amino acid skeleton is suitable for the above objects. The present invention was made based on this finding. More specifically, the present invention relates to a pyridine ring-containing benzoxazinone derivative represented by general formula (1) or a pharmaceutically acceptable salt or tautomer thereof: 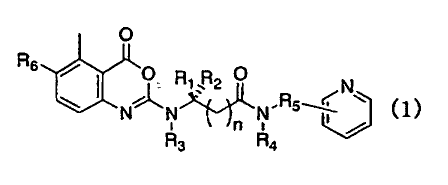 wherein R₁ and R₂ are independently selected from the group consisting of a hydrogen atom, alkyl, cycloalkyl, cycloalkylalkyl, haloalkyl, guanidinylalkyl, carboxyalkyl, hydroxyalkyl, alkoxyalkyl, aralkoxyalkyl, alkylthioalkyl, alkylsulfinylalkyl, alkylsulfonylalkyl, aryl, heterocyclyl, phenylalkyl, heterocyclylalkyl, and 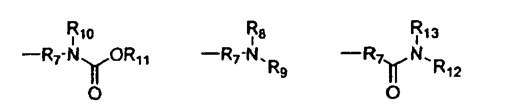 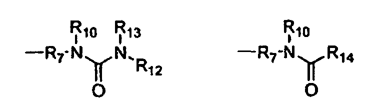 or R₁ and R₂ together form cycloalkyl such as R₁CR₂; R₃ is selected from the group consisting of a hydrogen atom, alkyl, alkylaminoalkyl, aralkyl, and heterocyclylalkyl or R₁ and R₃ or R₂ and R₃ together form 5- to 7-membered saturated or partially saturated heterocyclyl; n is 0 or 1; R₄ is selected from the group consisting of a hydrogen atom, alkyl, alkenyl, cycloalkyl, cycloalkylalkyl, aryl, heterocyclyl, aralkyl, heterocyclylalkyl, alkylaminoalkyl, and heterocyclylalkyl; R₅ is alkylene or forms 5- to 7-membered heterocyclyl such as -NR₄R₅ together with R₄; R₆ is selected from the group consisting of a hydrogen atom, halo, alkyl, and 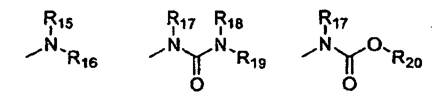 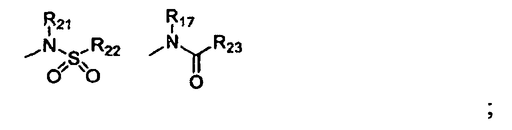 R₇ is alkylene; R₈, R₉, R₁₂, and R₁₃ are independently selected from the group consisting of a hydrogen atom, alkyl, cycloalkyl, cycloalkylalkyl, aminoalkyl, alkylaminoalkyl, hydroxyalkyl, alkoxyalkyl, aralkoxyalkyl, aryl, heterocyclyl, aralkyl, and heterocyclylalkyl, R₈ and R₉ together form 5- to 7-membered heterocyclyl such as -NR₈R₉, or R₁₂ and R₁₃ together form 5- to 7-membered heterocyclyl such as -NR₁₂R₁₃; R₁₀ is selected from the group consisting of a hydrogen atom, alkyl, and aralkyl; R₁₁ is selected from the group consisting of a hydrogen atom, alkyl, and aralkyl or R₁₁ is selected from the group consisting of alkyl, cycloalkyl, cycloalkylalkyl, alkylaminoalkyl, aralkoxyalkyl, alkoxyalkyl, aryl, aralkyl, heterocyclyl, and heterocyclylalkyl; R₁₄ is selected from the group consisting of a hydrogen atom, alkyl, cycloalkyl, cycloalkylalkyl, alkylaminoalkyl, aminoalkyl, carboxyalkyl, aminocarbonylalkyl, hydroxyalkyl, aralkoxyalkyl, alkoxyalkyl, aryl, aralkyl, heterocyclyl, and heterocyclylalkyl; R₁₅ and R₁₆ are independently selected from the group consisting of a hydrogen atom, alkyl, cycloalkyl, cycloalkylalkyl, aryl, aralkyl, heterocyclyl, heterocyclylalkyl, alkylaminoalkyl, aralkylaminoalkyl, alkoxyalkyl, and aralkoxyalkyl or R₁₅ and R₁₆ together form 5- to 7-membered heterocyclyl such as -NR₁₅R₁₆; R₁₇ and R₂₁ are independently selected from the group consisting of a hydrogen atom, alkyl, and aralkyl; R₁₈ and R₁₉ are independently selected from the group consisting of a hydrogen atom, alkyl, cycloalkyl, cycloalkylalkyl, haloalkyl, aminoalkyl, alkylaminoalkyl, carboxyalkyl, aminocarbonylalkyl, alkylaminocarbonylalkyl, hydroxyalkyl, alkoxyalkyl, aralkoxyalkyl, alkylsulfonylalkyl, alkylsulfinylalkyl, alkylthioalkyl, aryl, aralkyl, aralkenyl, heterocyclyl, and heterocyclylalkyl or R₁₈ and R₁₉ together form 5- to 7-membered heterocyclyl such as -NR₁₈R₁₉; R₂₀ is selected from the group consisting of alkyl, haloalkyl, alkylaminoalkyl, carboxyalkyl, aminocarbonylalkyl, alkylaminocarbonylalkyl, aralkoxyalkyl, alkoxyalkyl, cycloalkyl, cycloalkylalkyl, alkylsulfonylalkyl, alkylsulfinylalkyl, alkylthioalkyl, aryl, aralkyl, heterocyclyl, and heterocyclylalkyl; R₂₂ is selected from the group consisting of amino, alkyl, alkylamino, alkylaminoalkyl, and aryl; R₂₃ is selected from the group consisting of a hydrogen atom, alkyl, cycloalkyl, cycloalkylalkyl, haloalkyl, aminoalkyl, alkylaminoalkyl, carboxyalkyl, aminocarbonylalkyl, alkylaminocarbonylalkyl, hydroxyalkyl, alkoxyalkyl, aralkoxyalkyl, alkylsulfonylalkyl, alkylsulfinylalkyl, alkylthioalkyl, aryl, aralkyl, aralkenyl, heterocyclyl, heterocyclylalkyl, and 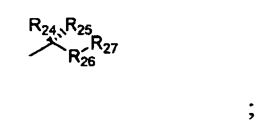 R₂₄ and R₂₅ are independently selected from the group consisting of a hydrogen atom, alkyl, cycloalkyl, cycloalkylalkyl, haloalkyl, guanidinylalkyl, carboxyalkyl, hydroxyalkyl, alkoxyalkyl, aralkoxyalkyl, alkylthioalkyl, alkylsulfinylalkyl, alkylsulfonylalkyl, aryl, heterocyclyl, aralkyl, heterocyclylalkyl, and 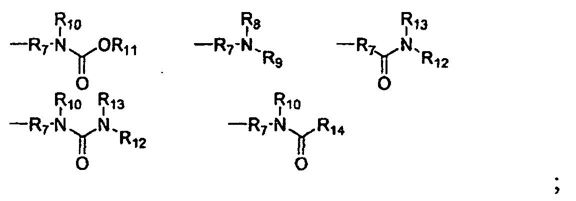 R₂₆ and R₂₈ are independently alkylene; and R₂₇ is selected from the group consisting of a hydrogen atom, halo, alkyl, and

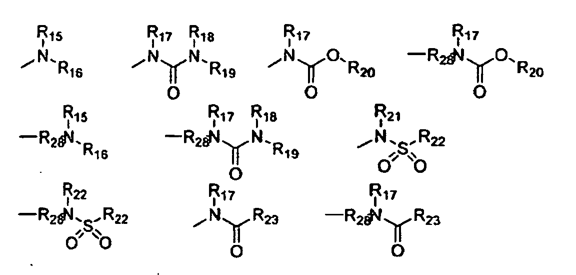

This description includes part or all of the contents as disclosed in the description and/or drawings of Japanese Patent Application No. 2001-379282, which is a priority document of the present application.

### Best Modes for Carrying out the Invention

It was verified that the compounds according to the present invention were particularly effective on Herpesviridae. Accordingly, these compounds are useful for treating infectious diseases such as HSV-1, HSV-2, CMV, VZV, Epstein-Barr virus, HHV6, HHV7, HHV8, pseudorabies, rhinotracheitis, and herpes B virus.

The present invention also includes a method for treating subjects infected with viruses using effective amounts of the compounds that can inhibit virus-specific proteases. Preferably, subjects are treated with assembly protease inhibitors. More preferably, subjects are treated with protease inhibitors encoded by the UL26 genes in the cases of HSV1 or HSV2, those encoded by the UL80 genes in the case of CMV, and those encoded by the VZV gene 33 in the case of VZV, such as the 4H-3,1-benzoxazin-4-one compound according to the present invention.

In addition to their usefulness in treating humans, these compounds are also useful for treating animals including, but not being limited to, pet animals and farm animals such as horses, dogs, cats, cattle, sheep, and pigs.

The compounds according to the present invention can be used in combination therapy simultaneously or sequentially with another type of substance having antiviral activities. The term "substance having antiviral activities" used herein refers to, for example, an agent for treating virus infections by suppressing virus multiplication through the inhibition of viral genome replication or virus particle formation or enhancing the resistance of the infected host against virus infections. An example of antiviral activity is antiherpes viral activity. An example of antiherpes viral activity is inhibitory activity on the DNA synthesis of herpes viruses. Specific examples of substances having antiviral activities include, but are not limited to, the following. Specifically, the compounds of the present invention can be used in combination with an antiviral agent such as ganciclovir, docosanol, trifluridine, foscarnet, ribavirn, eperudine, interferon, thymostimulin, CGP-16056 (Ciba-Geigy), sprofen, Efalith, ibuprofen piconol, ufeenamate, thymopentin, aciclovir, valaciclobir, edoxudine, famciclovir, idoxuridine, vidaravine, Epavir, zinc acetate, tromantadine, ridoxol, sorivudine, LC-9018 (Yakult Honsha Co., Ltd.), cidofovir, bromovinyldeoxyuridine, Lidakol, Stega Phamaceutical, a cytokine-releasing agent, CSL, ISCOM, penciclovir, Viraplex, THF (Pharmacia Upjohn), BIRR-4 (Behringer Ingelheim), NIH peptide T, Virend, zinc glycerolate, Pharmaprojects No 6645 (Pharmacia), valganciclovir, brivudine, viracol, viraplex, histamine dihydrochloride, vaccine preparations, HSV treatment (SLA Pharma), ME-609 or MIV-609 (Medivir), NVTL-001 (Novactyl), AG-701 (Antigenics), HSV inhibitors (Bayer), A-5021 (Ajinomoto Co., Inc.), AV-100 (Ajinomoto Co., Inc.), Pharmaprojects No. 6595 (GlaxoSmithKline), antisense preparations, T-611 (Tularik), CMV cell therapy (Targeted Genetics), fomivirsen sodium, mizoribine, interferon preparations, resiquimod, or lobucavir. Preferably, the compounds of the present invention can be used in combination with an antiviral agent such as aciclovir, penciclovir, valaciclovir, famciclovir, ganciclovir, valganciclovir, foscarnet, cidofovir, vidaravine, or Pharmaprojects No. 6645 (Pharmacia). More preferably, the compounds of the present invention can be used in combination with an antiviral agent such as aciclovir, penciclovir, valaciclovir, famciclvir, ganciclovir, or valganciclovir.

The term "combination therapy" refers to the use of the compound represented by general formula (1) according to the present invention in combination with another type of agent. A therapeutic regimen that provides beneficial effects through the combined use of these agents can lead to, for example, improved therapeutic effects, enhanced safety, and lowered frequency of the development of viruses resistant thereto. This includes simultaneous and sequential administration of these agents, such as administration of a single capsule containing given amounts of these active agents or several separate capsules each containing an agent and administration in different forms, i.e., where one agent is parenterally administered while another is orally administered.

A preferable group of compounds is a compound represented by general formula (1) or a pharmaceutically acceptable salt or tautomer thereof, wherein R₁ and R₂ are independently selected from the group consisting of a hydrogen atom, lower alkyl, phenylalkyl, cyclohexylmethyl, and heterocyclylmethyl or R₁ and R₂ together form lower cycloalkyl such as R₁CR₂, R₃ is selected from the group consisting of a hydrogen atom and lower alkyl, R₄ is selected from the group consisting of a hydrogen atom and lower alkyl, and R₅ is lower alkylene or forms 5- or 6-membered heterocyclyl together with R₄.

An example of a further preferable group of compounds is a compound represented by general formula (1) or a pharmaceutically acceptable salt or tautomer thereof, wherein R₁ and R₂ are independently selected from the group consisting of a hydrogen atom, methyl, benzyl, and phenylethyl or R₁ and R₂ together form cyclopropyl, cyclopentyl, or cyclohexyl.

A group of particularly preferable specific compounds and pharmaceutically acceptable salts thereof are shown below:
(5-methyl-2-{1-methyl-1-[methyl-(2-pyridin-2-yl-ethyl)-carbamoyl]-ethylamino}-4-oxo-4H-benzo[d][1,3]oxazin-6-yl)-carbamic acid tert-butyl ester;
(5-methyl-2-{1-methyl-1-[methyl-(2-pyridin-3-yl-ethyl)carbamoyl]-ethylamino}-4-oxo-4H-benzo[d][1,3]oxazin-6-yl)-carbamic acid tert-butyl ester;
(5-methyl-4-oxo-2- {2-phenyl-1S-[(pyridin-2-ylmethyl)-carbamoyl]-ethylamino}-4H-benzo[d][1,3]oxazin-6-yl)-carbamic acid tert-butyl ester;
(5-methyl-2-{1-[methyl-1S-(2-pyridin-2-yl-ethyl)-carbamoyl]-2-phenyl-ethylamino}-4-oxo-4H-benzo[d][1,3]oxazin-6-yl)-carbamic acid tert-butyl ester;
[5-methyl-2-(methyl-{2-phenyl-1S-[(pyridin-2-ylmethyl)-carbamoyl]-ethyl}-amino)-4-oxo-4H-benzo[d][1,3]oxazin-6-yl]-carbamic acid tert-butyl ester;
{5-methyl-2-[1S-(methyl-pyridin-2-yl-methyl-carbamoyl)-3-phenyl-propylamino]-4-oxo-4H-benzo[d][1,3][oxazin-6-yl}-carbamic acid tert-butyl ester;
{5-methyl-2-[1S-(methyl-pyridin-2-ylmethyl-carbamoyl)-ethylamino]-4-oxo-4H-benzo[d][1,3]oxazin-6-yl}-carbamic acid tert-butyl ester;
{5-methyl-2-[1-methyl-1-(methyl-pyridin-2-ylmethyl-carbamoyl)-ethylamino]-4-oxo-4H-benzo[d][1,3]oxazin-6-yl}-carbamic acid tert-butyl ester;
(5-methyl-2- {1S-[methyl-(2-pyridin-3-yl-ethyl)-carbamoyl]-ethylamino}-4-oxo-4H-benzo[d][1,3]oxazin-6-yl)-carbamic acid tert-butyl ester;
{5-methyl-2-[1-(methyl-pyridin-3-ylmethyl-carbamoyl)-cyclohexylamino]-4-oxo-4H-benzo[d][1,3]oxazin-6-yl}-carbamic acid tert-butyl ester; and
(5-methyl-2-{1-[methyl-(2-pyridin-4-yl-ethyl)-carbamoyl]-cyclohexylamino}-4-oxo-4H-benzo[d][1,3]oxazin-6-yl)-carbamic acid tert-butyl ester.

When the term "alkyl" is used alone or in other terms such as "haloalkyl," "alkylsulfonyl," "alkoxyalkyl," "hydroxyalkyl," or "aralkyl," this term includes a straight-chain or branched group of 1 to about 20 carbon atoms, and preferably 1 to about 12 carbon atoms. More preferable alkyl is "lower alkyl" of 1 to about 10 carbon atoms. Most preferable lower alkyl has 1 to about 6 carbon atoms. Examples thereof include methyl, ethyl, n-propyl, isopropyl, n-butyl, isobutyl, sec-butyl, tert-butyl, pentyl, isoamyl, and hexyl.

The term "alkylene" includes a straight-chain or branched group of 1 to about 20 carbon atoms, and preferably 1 to about 12 carbon atoms, that are substituted with other functional groups at both ends. More preferable alkylene is "lower alkylene" of 1 to about 10 carbon atoms. Most preferable lower alkylene has 1 to about 6 carbon atoms. Examples thereof include methylene, ethylene, n-propylene, isopropylene, n-butylene, isobutylene, sec-butylene, n-pentylene, and n-hexylene.

The term "alkenyl" includes a straight-chain or branched group containing at least one carbon-carbon double bond and 2 to about 20 carbon atoms, and preferably 2 to about 12 carbon atoms. It should be noted that this double bond is not present at a binding site on the aforementioned group. More preferable alkenyl is "lower alkenyl" of 2 to about 6 carbon atoms. Examples thereof include ethenyl, n-propenyl, and butenyl. The term "halo" refers to a halogen such as fluorine, chlorine, bromine, or iodine.

The term "haloalkyl" includes a group, where at least one alkyl carbon atom thereof is substituted with "halo" as defined above. Specific examples include monohaloalkyl, dihaloalkyl, and polyhaloalkyl. For example, monohaloalkyl can contain an iodo, bromo, chloro, or fluoro atom therein. Dihaloalkyl and polyhaloalkyl can contain combinations of two or more identical or different halo atoms. The term "lower haloalkyl" includes a group of 1 to 6 carbon atoms. Examples of haloalkyl include fluoromethyl, difluoromethyl, trifluoromethyl, chloromethyl, dichloromethyl, trichloromethyl, trichloromethyl, pentafluoroethyl, heptafluoropropyl, difluorochloromethyl, dichlorofluoromethyl, difluoroethyl, difluoropropyl, dichloroethyl, and dichloropropyl.

The term "hydroxyalkyl" includes straight-chain or branched alkyl of 1 to about 10 carbon atoms and at least one thereof may be substituted with at least one hydroxyl. More preferable hydroxyalkyl is "lower hydroxyalkyl" that contains 1 to 6 carbon atoms and at least one hydroxyl. Examples thereof include hydroxymethyl, hydroxyethyl, hydroxypropyl, hydroxybutyl, and hydroxyhexyl.

The terms "alkoxy" and "alkoxyalkyl" independently include a straight-chain or branched oxy-containing group of 1 to about 10 carbon atoms at the alkyl moiety. More preferable alkoxy is "lower alkoxy" of 1 to 6 carbon atoms. Examples thereof include methoxy, ethoxy, propoxy, butoxy, and tert-butoxy.

The term "alkoxyalkyl" includes alkyl that has two or more alkoxys bound thereto, i.e., alkyl that forms monoalkoxyalkyl and dialkoxyalkyl. More preferable alkoxyalkyl is "lower alkoxyalkyl" that contains 1 to 6 carbon atoms and 1 or 2 alkoxys. Examples thereof include methoxymethyl, methoxyethyl, ethoxyethyl, methoxybutyl, and methoxypropyl. The term "alkoxy" or "alkoxyalkyl" may be further substituted with at least one halo atom such as fluoro, chloro, or bromo, and it may form "haloalkoxy" or "haloalkoxyalkyl." More preferable haloalkoxy is "lower haloalkoxy" that contains 1 to 6 carbon atoms and one or more halos. Examples thereof include fluoromethoxy, chloromethoxy, trifluoromethoxy, trifluoroethoxy, fluoroethoxy, and fluoropropoxy.

The term "aryl" refers to a carbocyclic aromatic group containing 1, 2, or 3 rings alone or in combination. These rings can be together bound via side chain or condensed.

The term "aryl" includes an aromatic group such as phenyl, naphthyl, tetrahydronaphthyl, indanyl, and biphenylyl. The aryl moiety can have at least one substituent at a substitutable position independently selected from the group consisting of alkyl, aralkyl, alkoxyalkyl, alkylaminoalkyl, carboxyalkyl, alkoxycarbonylalkyl, aminocarbonylalkyl, alkoxy, aralkoxy, heterocyclylalkoxy, alkylaminoalkoxy, carboxyamino, carboxyaminoalkyl, carboxyaminoaralkyl, amino, halo, nitro, alkylamino, alkylcarbonylamino, arylcarbonylamino, alkoxycarbonylamino, aralkoxycarbonylamino, aminocarbonylamino, alkylaminocarbonylamino, alkylsulfonylamino, arylsulfonylamino, acyl, cyano, carboxyl, aminocarbonyl, alkoxycarbonyl, and aralkoxycarbonyl.

The term "heterocyclyl" or "heterocyclic" includes saturated, partially saturated, and unsaturated hetero atom-containing ring-forming groups. A hetero atom can be selected from the group consisting of nitrogen, sulfur, and oxygen. Examples of saturated heterocyclic groups include saturated 5- to 7-membered heteromonocyclic groups containing 1 to 4 nitrogen atoms (e.g., pyrrolidinyl, imidazolidinyl, piperidinyl, piperazinyl, tropanyl, or homotropanyl), saturated 5- to 7-membered heteromonocyclic groups containing 1 or 2 oxygen atoms and 1 to 3 nitrogen atoms (e.g., morpholyl), and saturated 5- to 7-membered heteromonocyclic groups containing 1 or 2 sulfur atoms and 1 to 3 nitrogen atoms (e.g., thiazolydinyl). Examples of partially saturated heterocyclic groups include dihydrothienyl, dihydropyranyl, oxazolinyl, dihydrofuryl, and dihydrothiazolyl.

Examples of unsaturated heterocyclic groups that are also represented by the term "heteroaryl" include: unsaturated 5- to 7-membered heteromonocyclic groups containing 1 to 4 nitrogen atoms (e.g., pyrrolyl, pyrrolynyl, imidazolyl, pyrazolyl, pyridyl, pyrimidyl, azepinyl, pyrazinyl, pyridazinyl, triazolyl (such as 4H-1,2,4-triazolyl, 1H-1,2,3-triazolyl, or 2H-1,2,3-triazolyl), or tetrazolyl (such as 1H-tetrazolyl or 2H-tetrazolyl)); unsaturated condensed heterocyclic groups containing 1 to 5 nitrogen atoms (e.g., indolyl, isoindolyl, indolydinyl, benzimidazolyl, quinolyl, isoquinolyl, indazolyl, benzotriazolyl, or tetrazoropyridazinyl (such as tetrazoro[1,5-b]pyridazinyl)); unsaturated 3- to 6-membered heteromonocyclic groups containing an oxygen atom (e.g., pyranyl or furyl); unsaturated 5- to 7-membered heteromonocyclic groups containing a sulfur atom (e.g., thienyl); unsaturated 5- to 7-membered heteromonocyclic groups containing 1 or 2 oxygen atoms and 1 to 3 nitrogen atoms (e.g., oxazolyl, isooxazolyl, or oxadiazolyl (such as 1,2,4-oxadiazolyl, 1,3,4-oxadiazolyl, or 1,2,5-oxadiazolyl)); unsaturated condensed heterocyclic groups containing 1 or 2 oxygen atoms and 1 to 3 nitrogen atoms (e.g., benzoxazolyl or benzooxadiazolyl); unsaturated 5- to 7-membered heteromonocyclic groups containing 1 or 2 sulfur atoms and 1 to 3 nitrogen atoms (e.g., thiazolyl or thiadiazolyl (such as 1,2,4-thiadiazolyl, 1,3,4-thiadiazolyl, or 1,2,5-thiadiazolyl)); and unsaturated condensed heterocyclic groups containing 1 or 2 sulfur atoms and I to 3 nitrogen atoms (e.g., benzothiazolyl or benzothiadiazolyl). This term also includes a group wherein the heterocyclic group thereof is condensed with aryl. Examples of such condensed bicyclic groups include benzofuryl and benzothienyl.

The above "heterocyclyl" can have, at a substitutable position, at least one substituent selected from the group consisting of alkyl, aralkyl, alkoxyalkyl, alkylaminoalkyl, carboxyalkyl, alkoxycarbonylalkyl, aminocarbonylalkyl, alkoxy, aralkoxy, alkylaminoalkoxy, aminocarboxy, alkylaminocarboxy, aralkylaminocarboxy, amino, halo, nitro, alkylamino, alkylcarbonylamino, arylcarbonylamino, alkoxycarbonylamino, aralkoxycarbonylamino, aminocarbonylamino, alkylaminocarbonylamino, alkylsulfonylamino, arylsulfonylamino, acyl, cyano, carboxy, aminocarbonyl, alkoxycarbonyl, and aralkoxycarbonyl. 5- or 6-membered heteroaryl is preferable.

The term "cycloalkyl" includes a group of 3 to 10 carbon atoms. More preferable cycloalkyl is "lower cycloalkyl" of 3 to 7 carbon atoms. Examples thereof include cyclopropyl, cyclobutyl, cyclopentyl, cyclohexyl, and cycloheptyl.

The term "cycloalkylalkyl" includes cycloalkyl as defined above, which is bound to alkyl of 1 to 10 carbon atoms. More preferable cycloalkylalkyl is "lower cycloalkylalkyl" containing alkyl of 1 to 6 carbon atoms. Examples thereof include cyclopropylpropyl, cyclobutylethyl, cyclopentylmethyl, and cyclohexylmethyl.

The term "mercaptoalkyl" includes a group having free -SH that is bound to straight-chain or branched alkyl as defined above. Examples of such mercaptoalkyl include mercaptomethyl, mercaptopropyl, and mercaptohexyl.

The term "alkylthio" includes a group containing straight-chain or branched alkyl of 1 to about 10 carbon atoms bound to a divalent sulfur atom. More preferable alkylthio is "lower alkylthio" having alkyl of 1 to 6 carbon atoms. Examples of such lower alkylthio include methylthio, ethylthio, propylthio, butylthio, and hexylthio.

The term "alkylthioalkyl" includes alkylthio bound to alkyl. More preferable alkylthioalkyl is "lower alkylthioalkyl" having alkyl of 1 to 6 carbon atoms. An example thereof is methylthiomethyl.

The term "alkylsulfinyl" includes a group having straight-chain or branched alkyl of 1 to 10 carbon atoms bound to divalent -S(=O)-.

More preferable alkylsulfinyl is "lower alkylsulfinyl" of 1 to 6 carbon atoms. Examples of such lower alkylsulfinyl include methylsulfinyl, ethylsulfinyl, butylsulfinyl, and hexylsulfinyl. The term "sulfonyl" refers to divalent -SO₂- regardless of whether it is used alone or in conjunction with another term, such as alkylsulfonyl. "Alkylsulfonyl" includes alkyl as defined above that is bound to sulfonyl. More preferable alkylsulfonyl is "lower alkylsulfonyl" of 1 to 6 carbon atoms. Examples of such lower alkylsulfonyl include methylsulfonyl, ethylsulfonyl, and propylsulfonyl. This "alkylsulfonyl" may also be substituted with at least one halo atom such as fluoro, chloro, or bromo, and it may form "haloalkylsulfonyl." More preferable haloalkylsulfonyl is "lower haloalkylsulfonyl" having at least one halo atom bound to lower alkylsulfonyl as defined above. Examples of such lower haloalkylsulfonyl include fluoromethylsulfonyl, trifluoromethylsulfonyl, and chloromethylsulfonyl.

The term "arylsulfonyl" includes aryl as defined above that is bound to sulfonyl. An example thereof is phenylsulfonyl.

The terms "sulfamyl," "aminosulfonyl," and "sulfonamidyl" independently refer to -SO₂NH₂. The term "acyl" refers to a group provided by a residue after hydroxyl is removed from organic acid. Examples of such acyl include formyl, alkanoyl, and aroyl.

The term "alkylsulfinylalkyl" includes a group containing alkylsulfinyl as defined above that is bound to alkyl. More preferable alkylsulfinylalkyl is "lower alkylsulfinylalkyl" of 1 to 6 carbon atoms. Examples of such lower alkylsulfinylalkyl include methylsulfinylethyl, ethylsulfinylmethyl, butylsulfinylethyl, and methylsulfinylmethyl.

The term "alkylsulfonylalkyl" includes alkylsulfonyl as defined above that is bound to alkyl as defined above. More preferable alkylsulfonylalkyl is "lower alkylsulfonylalkyl" of 1 to 6 carbon atoms. Examples of such lower alkylsulfonylalkyl include methylsulfonylmethyl, ethylsulfonylethyl, and methylsulfonylethyl. The term "carboxy" or "carboxyl" refers to -CO₂H regardless of whether it is used alone or in conjunction with another term, such as "carboxyalkyl." The term "carbonyl" refers to -(C=O)- regardless of whether it is used alone or in conjunction with another term, such as "alkoxycarbonyl."

The term "alkoxycarbonyl" refers to a group containing alkoxy as defined above that is bound to carbonyl via an oxygen atom. Preferable "lower alkoxycarbonyl" includes alkoxy of 1 to 6 carbon atoms. Examples of such "lower alkoxycarbonyl" ester include methoxycarbonyl, ethoxycarbonyl, propoxycarbonyl, butoxycarbonyl, and hexyloxycarbonyl, that have substituents or that are unsubstituted.

The term "aralkyl" includes aryl-substituted alkyl. Preferable aralkyl is "lower aralkyl" having aryl that is bound to alkyl of 1 to 6 carbon atoms. Examples thereof include benzyl, diphenylmethyl, triphenylmethyl, phenylethyl, and diphenylethyl. The aryl in the above aralkyl may be further substituted as defined above.

The term "aralkenyl" includes aryl-substituted alkenyl. Preferable aralkenyl is "lower phenylaralkyl" having phenyl that is bound to alkyl of 1 to 6 carbon atoms. Examples thereof include phenylethenyl and phenylpropenyl. Aryl in the above aralkyl may be further substituted as defined above.

The terms "benzyl" and "phenylmethyl" can be reciprocally exchanged. The term "alkylcarbonyl" includes a group having alkyl as defined above that is bound to carbonyl. More preferable alkylcarbonyl is "lower alkylcarbonyl" of 1 to 6 carbon atoms. Examples thereof include methylcarbonyl and ethylcarbonyl.

The term "alkoxycarbonylalkyl" includes a group having "alkoxycarbonyl" as defined above that is substituted with alkyl. More preferable alkoxycarbonylalkyl is "lower alkoxycarbonyl," and this group has lower alkoxycarbonyl as defined above that is bound to 1 to 6 carbon atoms. An example of such lower alkoxycarbonylalkyl is methoxycarbonylmethyl. The term "haloalkylcarbonyl" includes a group having haloalkyl as defined above that is bound to carbonyl. A more preferable group is "lower haloalkylcarbonyl" in which lower haloalkyl as defined above is bound to carbonyl.

The term "carboxyalkyl" includes a group having carboxy as defined above that is bound to alkyl. Alkanoyl may be substituted or unsubstituted. Examples thereof include formyl, acetyl, propionyl, butyryl, isobutyryl, valeryl, isovaleryl, pivaloyl, hexanoyl, and trifluoroacetyl, with formyl, acetyl, propionyl, trifluoroacetyl, or the like being preferable.

The term "heterocyclylalkyl" includes heterocycle-substituted alkyl. More preferable heterocyclylalkyl is "lower heterocyclylalkyl," and this group has a 5- or 6-membered heterocycle that is bound to lower alkyl of 1 to 6 carbon atoms. Examples thereof include pyrrolidinylmethyl, piperidinylmethyl, molpholinylmethyl, piperazinylmethyl, oxazolylmethyl, oxazolylethyl, oxazolinylmethyl, oxazolinylethyl, indolylethyl, indolylmethyl, pyridylmethyl, quinolylmethyl, thienylmethyl, furylethyl, and quinolylethyl. Heterocyclyl that is present in this heterocyclylalkyl may be further substituted as defined above. The term "aryloxy" includes aryl as defined above that is bound to an oxygen atom.

Aryl that is present in this aryloxy may be further substituted as defined above. An example thereof is phenoxy. The term "aralkoxy" includes oxygen-containing aralkyl that is bound to another group via an oxygen atom.

The term "aralkoxyalkyl" includes alkyl having at least one aralkoxy that is bound to alkyl, that is, a group forming monoaralkyloxyalkyl and diaralkyloxyalkyl. This "aralkoxy" or "aralkoxyalkyl" may further have a substituent at its ring portion of aryl. More preferable aralkoxyalkyl is "lower aralkoxyalkyl" having alkoxy that is bound to 1 to 6 carbon atoms. An example of lower aralkoxyalkyl is benzyloxymethyl.

The term "aminoalkyl" includes alkyl that is substituted with amino. More preferable aminoalkyl is "lower aminoalkyl" of 1 to 6 carbon atoms. Examples thereof include aminomethyl, aminoethyl, and aminobutyl.

The term "guanidinoalkyl" refers to guanidino [-C=NH₂(NH₂)₂] that is bound to alkyl as defined above. More preferable alkylamino is "lower alkylamino" in which one or two alkyls that contains 1 to 6 carbon atoms are bound to a nitrogen atom.

The term "alkylamino" refers to amino that is substituted with one or two alkyls. More preferable alkylamino is "lower alkylamino" in which one or two alkyls that contains 1 to 6 carbon atoms are bound to a nitrogen atom of amine. Suitable "lower alkylamino" can be mono or dialkylamino, such as N-methylamino, N-ethylamino, N,N-dimethylamino, or N,N-diethylamino.

The term "alkylaminoalkyl" refers to alkylamino as defined above that is bound to alkyl. More preferable alkylaminoalkyl is bound to lower alkylamino as defined above, and it is "lower alkylaminoalkyl" of 1 to 6 carbon atoms. Suitable "lower alkylaminoalkyl" is mono or dialkylaminoalkyl, such as N-methylaminomethyl, N-ethylaminomethyl, N,N-dimethylaminomethyl, N,N-dimethylaminoethyl, or N,N-dimethylaminopropyl.

The term "dialkylaminoalkyl" includes a group in which the alkyl crosslinking moiety may be substituted with alkylsulfonyl, alkoxy, aralkoxy, heterocyclyl, or aryl.

The term "alkylaminoalkoxy" refers to alkylamino as defined above that is bound to alkoxy. Suitable "alkylaminoalkoxy" is mono or dialkylaminoalkoxy, such as N-methylaminomethoxy, N-ethylaminomethoxy, N,N-dimethylaminomethoxy, N,N-dimethylaminoethoxy, or N,N-dimethylaminopropoxy.

The term "alkylaminocarbonyl" includes alkylamino as defined above that is bound to carbonyl. More preferable alkylaminocarbonyl is "lower alkylaminocarbonyl" having lower alkylamino as defined above that is bound to carbonyl. Examples thereof include N-methylaminocarbonyl and N,N-dimethylaminocarbonyl.

The term "arylamino" refers to amino that is substituted with 1 or 2 aryls, such as N-phenylamino. In "arylamino," the ring portion of aryl may be further substituted.

The terms "N-arylaminoalkyl" and "N-aryl-N-alkylaminoalkyl" independently refer to amino, which is substituted with 1 aryl or with 1 aryl and I alkyl and has amino that is bound to alkyl. More preferable arylaminoalkyl is "lower arylaminoalkyl" containing arylamino that is bound to 1 to 6 carbon atoms. Examples thereof include N-phenylaminomethyl and N-phenyl-N-methylaminomethyl.

The term "aminocarbonyl" refers to amide represented by formula: -C(=O)NH₂.

The term "aminocarbonylalkyl" refers to carbonylalkyl that is bound to alkyl. Preferable "lower aminocarbonylalkyl" has lower aminocarbonyl as defined above that is bound to alkyl of 1 to 6 carbon atoms.

The term "alkylaminocarbonylalkyl" refers to aminocarbonyl that is substituted with 1 or 2 alkyls and bound to alkyl. Preferable "lower alkylaminocarbonylalkyl" has lower alkylaminocarbonyl as defined above that is bound to alkyl of 1 to 6 carbon atoms.

The term "aryloxyalkyl" refers to alkyl that is bound to a divalent oxygen atom having at least 1 aryloxy and aryl bound to alkyl; that is, a group that forms monoaryloxyalkyl and diaryloxyalkyl. More preferable aryloxyalkyl is "lower aryloxyalkyl" having aryloxy that is bound to 1 to 6 carbon atoms. An example thereof is phenoxymethyl. The term "amino acid residue" refers to all of naturally occurring α-, β-, and γ-aminocarboxylic acids (including D- and L-enantiomers thereof and racemic mixtures thereof), synthetic amino acids, and derivatives of these naturally occurring and synthetic amino acids. This amino acid residue is bound by nitrogen of the amino acid. Examples of naturally occurring amino acids within the scope of the present invention include, but are not limited to, alanine, arginine, asparagine, aspartic acid, cysteine, cystine, glutamic acid, glutamine, glycine, histidine, isoleucine, leucine, lysine, methionine, ornithine, phenylalanine, proline, serine, threonine, thyroxine, tryptophan, thyrosin, valine, β-alanine, and γ-aminobutyric acid.

The amino acid derivative within the scope of the present invention includes, but is not limited to, acid ester and amide, and amino acid containing protected and denatured carboxylic acids, including protected amine and substituted phenyl ring. Examples thereof include, but are not limited to, alkyl, alkoxy, and halo-substituted thyrosine and phenylalanine.

The present invention includes a pharmaceutical composition that contains a therapeutically effective amount of the compound represented by general formula 1 in combination with at least one pharmaceutically acceptable carrier, an adjuvant, a diluent, or, if necessary, another substance having antiviral activity.

The present invention also includes a method for treating and preventing virus infections, especially herpesviridae infections, of a subject. In this method, a subject infected with herpes is treated with a pharmaceutical composition containing at least a therapeutically effective amount of the compound represented by general formula 1.

The present invention also includes a method for inhibiting viral protease. This method comprises the administration of a therapeutically effective amount of the compound represented by general formula 1.

A group of compounds represented by general formula 1 includes a stereoisomer and a tautomer thereof. The compound according to the present invention can contain at least one asymmetric carbon atom. Thus, it can be present in the form of an enantiomer, a racemate thereof, or a nonracemic mixture thereof. Accordingly, several types of the compounds of the present invention can be present as racemic mixtures. These are also within the scope of the present invention. An enantiomer can be obtained by dividing a racemic mixture through the formation of diastereomeric salt by processing with, for example, optically active acid or base in accordance with a conventional technique. Examples of suitable acids include tartaric, diacetyl tartaric, dibenzoyl tartaric, ditoluoyl tartaric, and camphor sulfonic acids. Subsequently, diastereomer mixtures are separated by crystallization, and optically active bases are then released from these salts. The other method for separating an enantiomer involves the use of a chiral chromatography column that is adequately selected for the maximal separation of the enantiomer. Another method that can be used involves the synthesis of covalent diastereomeric molecules in which an amine functional group, which is a precursor of the compound represented by general formula 1, is allowed to react with optically pure acid or isocyanate in an active state. Alternatively, diastereomeric derivatives can be produced by allowing a carboxyl functional group, which is a precursor of the compound represeted by general formula 1, to react with an optically pure amine base. The synthesized diastereomer can be separated by conventional techniques such as chromatography, distillation, crystallization, or sublimation, followed by hydrolysis. Thus, a pure compound can be released as an enantiomer. The optically active compound represented by general formula 1 can also be obtained by using an optically active starting material. These isomers can be in the form of free acid, free base, ester, or salt. Alternatively, other methods for dividing enantiomers that are known to persons skilled in the art can be used, for example, a method described in "Enantiomers, Racemate, and Resolutions," J. Jaqes, et al., John Wiley and Son, New York (1981).

A group of compounds represented by general formula 1 includes pharmaceutically acceptable salts thereof. The term "pharmaceutically acceptable salt" includes salts common to the formation of addition salts of free acid or base and to the formation of alkali metal salts. Salts are not particularly limited as long as they are pharmaceutically acceptable. Suitable pharmaceutically acceptable acid addition salts of the compound represented by general formula 1 can be produced from inorganic or organic acid. Examples of inorganic acids include hydrochloric, hydrobromic, hydriodic, nitric, carbonic, sulfuric, and phosphoric acids. Suitable organic acids can be selected from the group consisting of fatty, cyclic fatty, aromatic, fatty-aromatic, heterocyclic carboxylic, and sulfonic acids, which are classified as organic acids. Examples thereof include formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, stearic, cyclohexylaminosulfonic, arginic, β-hydroxybutyric, gallic, and galacturonic acids. Suitable pharmaceutically acceptable base addition salts of the compound represented by general formula 1 include metallic salts made from aluminum, calcium, lithium, magnesium, potassium, sodium, and zinc, and organic salts made from N,N'-dibenzylethylenediamine, choline, chloroprocaine, diethanolamine, ethylenediamine, meglumine (N-methylglucamine), and procaine. All of these salts can be prepared by conventional means from the corresponding compound represented by general formula I by allowing this compound to react with, for example, the appropriate acid or base.

A group of pharmaceutical compositions comprising at least one compound represented by general formula 1 in combination with at least one pharmaceutically acceptable nontoxic carrier, a diluent, and/or adjuvant (these are collectively referred to as "carrier" substances herein), and, if necessary, another substance having antiviral activity are within the scope of the present invention. The compound of the present invention can be administered by a suitable route of administration in a form of pharmaceutical composition that is suitable for such route in a dosage effective for the treatment of interest. These compounds and compositions can be administered, for example, orally, intravascularly, intraperitoneally, subcutaneously, intramuscularly, or topically. When the compound of the present invention is used as an agent for combination therapy, it may be placed in a container together with another antiviral agent in, for example, a powdery or liquid state. Occasionally, these agents may be stored in different containers and used simultaneously or sequentially.

The pharmaceutical composition of the present invention can be in the form of, for example, a tablet, capsule, suspension, or liquid for oral administration. The pharmaceutical composition is preferably prepared in a dosage unit containing a specific amount of active ingredient. Examples of such a dosage unit include a tablet and a capsule. An active ingredient can be administered by injection as a composition, and examples of a suitable carrier in the composition that can be used include brine, glucose, and water.

The dosage of the therapeutically active compound and the dosage regimen for treating a disease condition with the compounds and/or compositions of the present invention are affected by a variety of factors, including the age, weight, sex, and medical condition of the patient, the severity of the diseases, the route and the frequency of administration, and the types of compounds to be used. Thus, the dosage regimen may vary widely. The pharmaceutical composition can contain about 0.1 mg to 2,000 mg, preferably about 0.5 mg to 500 mg, and most preferably about 1 mg to 100 mg of active ingredients. The dose is suitably about 0.01 mg/kg to 100 mg/kg (per body weight), preferably about 0.1 mg/kg to about 50 mg/kg (per body weight), and most preferably about 1 mg/kg to 20 mg/kg (per body weight) per day. This dose can be administered, for example, in a single dose or up to 5 separate doses per day. When these agents are used as agents for combination therapy, the quantitative (molar) ratio of the active ingredient of the compound of the present invention represented by general formula 1 to that of another substance having antiviral activity can be 100:1 to 1:100, and is preferably 1:10 to 10:1.

In accordance with the purpose of treatment, the compound of the present invention is generally combined with at least one adjuvant that is suitable for the designated route of administration. In the case of oral administration, the compound is mixed with sodium salt and calcium salt of lactose, sucrose, starch powder, cellulose ester of alkanoic acid, cellulose alkyl ester, talc, stearic acid, magnesium stearate, magnesium oxide, phosphoric acid, and sulfuric acid, gelatin, gum arabic, sodium alginate, polyvinylpyrrolidone, and/or polyvinyl alcohol. The mixture is then made into tablets or encapsulated for the convenience of administration. Such capsules and tablets can contain a controlled release preparation, and can be provided as, for example, a dispersed system in hydroxyproplyl methylcellulose of an active compound. Preparations suitable for parenteral administration can be in the form of an aqueous, nonaqueous, or isotonic sterile parenteral solution or a suspension. The solution and the suspension can be prepared from sterile powders or granules containing at least one carrier or diluent for oral preparations mentioned above. The compound can be dissolved in water, polyethylene glycol, propylene glycol, ethanol, corn oil, cottonseed oil, peanut oil, sesame oil, benzyl alcohol, sodium chloride, and/or various types of buffers. Other adjuvants and manners of administration are widely and well known in the field of pharmaceuticals.

Preparations for infectious diseases of ophthalmic and other external tissues, such as the mouth and skin, are preferably applied as a topical ointment or cream containing, for example, 0.075% W/W to 30% W/W, preferably 0.2% W/W to 20% W/W, and most preferably 0.4% W/W to 15% W/W of an active ingredient (or active ingredients) in total. When prepared as an ointment, an active ingredient (or active ingredients) can be used in combination with a paraffin or water-miscible ointment base. Alternatively, an active ingredient (or active ingredients) can be formulated in a cream together with an oil-in-water cream base. If necessary, an aqueous phase of the cream base can contain, for example, polyhydric alcohol of at least 30% W/W, such as propylene glycol, butane-1,3-diol, mannitol, sorbitol, glycerin, polyethylene glycol, or a mixture thereof. Topical preparations preferably contain a compound that accelerates the absorption or penetration of an active ingredient (or active ingredients) through the skin or another affected area. Examples of such accelerators for penetration into skin include dimethyl sulfoxide and related analogous substances. The compound of the present invention can be administered using an apparatus for transdermal absorption. Topical administration may involve the use of a patch of a reservoir-type, porous membrane-type, or variation of solid matrix. In any case, an active agent is constantly supplied from a reservoir site or microcapsule via a membrane to an active agent-permeable adhesive that is in contact with the skin or mucous membrane of the patient. When the active agent is transdermally absorbed, a controlled and predetermined stream of the active agent is administered to the patient. In the case of mucrocapsules, the encapsulating agent is also capable of functioning as a membrane.

The oil phase in the emulsion according to the present invention can be made from known ingredients by a conventional technique. This oil phase may be simply comprised of an emulsifier, and it may be comprised of a mixture of at least one emulsifier with fat or oil or a mixture of at least one emulsifier with both fat and oil. Preferably, the oil phase contains a lipophilic emulsifier that functions as a stabilizer in combination with a hydrophilic emulsifier. The oil phase preferably contains both oil and fat. As a whole, an emulsifier (or emulsifiers) constitutes a so-called emulsified wax with or without a stabilizer (or stabilizers), this wax constitutes a so-called emulsified ointment base with oil and fat, and the latter forms an oil-dispersed phase of cream preparations. Specific examples of emulsifiers and emulsion stabilizers that are suitable for the preparation of the present invention include Tween 60, Span 80, cetostearyl alcohol, myristyl alcohol, glyceryl monostearate, and sodium lauryl sulfate.

The choice of suitable oils or fats for the preparation is based on the achievement of the cosmetic properties of interest, because the solubility of the active compound in most oils that are likely to be used in pharmaceutical emulsion formulations is very low. Therefore, the cream should preferably be a non-greasy, non-staining, and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate, or a blend of branched chain esters may be used. These may be used alone or in combination, depending on the properties required. Alternatively, high melting-point lipids, such as soft white paraffin and/or liquid paraffin, or other mineral oils, can be used.

As a suitable preparation for topical administration to eyes, an active ingredient is dissolved or suspended in a suitable carrier, especially an aqueous medium suitable for the active ingredient. An ingredient having antiviral activity is preferably present in such a preparation at a concentration level of 0.5% to 20% W/W, effectively of 0.5% to 10% W/W, and particularly effectively of about 1.5% W/W.

The compound of the present invention can be synthesized from a commercialized 2-aminobenzoic acid compound in accordance with the methods shown in formulae 13 to 18 below. In these formulae 13 to 18, substituents R₁ to R₂₈ are as defined in general formula 1 unless otherwise specified.

Formula 13 shows a representative process of synthesis.

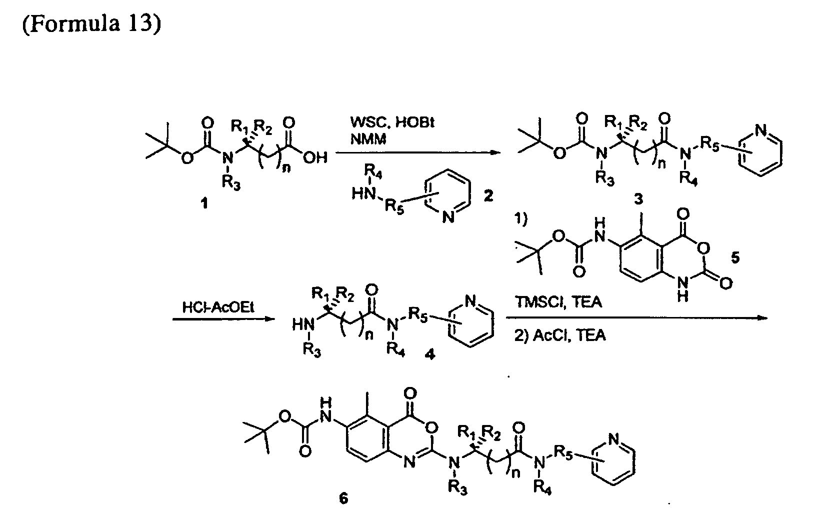

Boc-amino acid 1 is allowed to activate in the presence of 1-(3-dimethylaminopropyl)-3-ethylcarbodiimide hydrochloride (EDC, alternatively referred to as water soluble carbodiimide (WSC)), N-hydroxybenzotriazole (HOBt), and N-methylmorpholine (NMM). Subsequently, it is processed with corresponding pyridine ring-containing primary or secondary amine 2. Thus, a protected amide compound 3 is obtained. Boc can be separated by acid treatment using HCl-containing ethyl acetate, and free amine 4 can be obtained from an aqueous solution of sodium hydroxide. In a suspension of a known compound 5 in dichloromethane (DCM), triethylamine (TEA) and then trimethylsilyl chloride (TMSCI) are added to generate isocyanate, the solution of 4 in DCM prepared above is added to prepare urea carboxylic acid, and the product is washed with water. With the use of TEA and acetyl chloride (AcCl) thereafter, a compound 6 of interest can be provided by intramolecular ring-closure via mixed acid anhydride.

Parallel synthesis was carried out by the processes of synthesis shown in formulae 14 to 18 below. The process for synthesizing a compound in which R₆ is immobilized on BocNH is shown in formula 14 below.

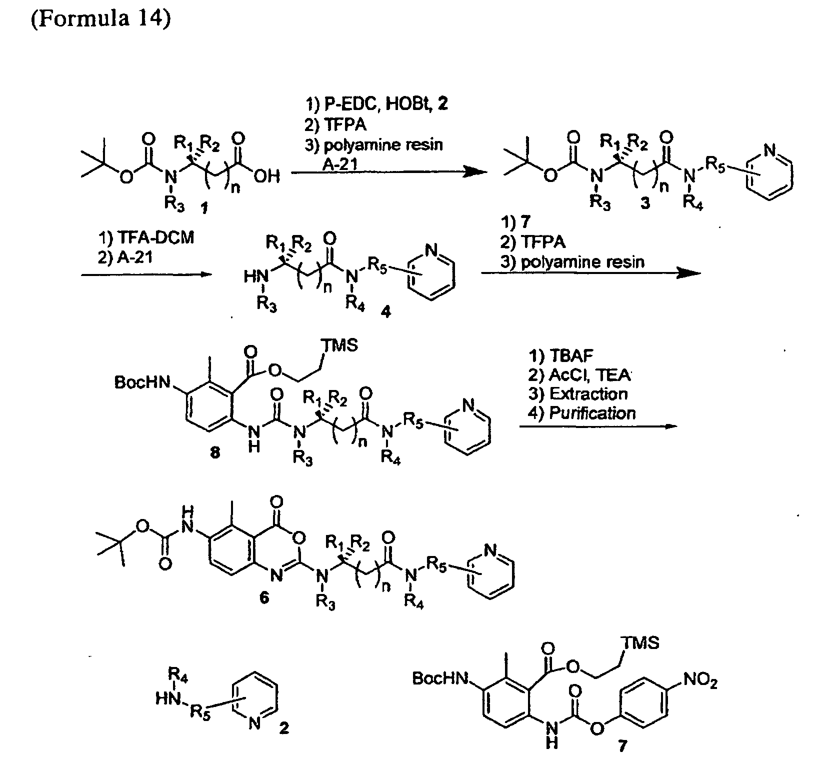

Boc-amino acid 1 is allowed to activate in the presence of polymer-1-(3-dimethylaminopropyl)-3-ethylcarbodiimide (P-EDC) and HOBt. Subsequently, it is processed with corresponding pyridine ring-containing primary or secondary amine 2. Thus, a protected amide compound 3 is obtained. The unreacted 2 is converted into carboxylic acid with the aid of tetrafluorophthalic anhydride (TFPA), and it can be removed with the use of a polyamine resin together with the unreacted 1, the activated 1, and HOBt. Boc can be separated by acid treatment using trifluoroacetic acid (TFA), and free amine 4 can be obtained with the use of the Amberlist A-21. A solution of a known compound 7 in DCM is then added to the solution of 4 in DCM. Thus, the urea body 8 of interest can be obtained. After the unreacted 4 and the aniline body, which is a precursor of 7, are converted into carboxylic acid with the aid of TFPA, excess amounts of reagents can be removed with the use of a polyamine resin. Urea carboxylic acid is prepared with the aid of tetrabutylammonium chloride, and TFA and AcCl are used. Thus, a compound 6 of interest can be provided by intramolecular ring-closure via mixed acid anhydride. Compounds can be purified using a parallel silica gel column after parallel extraction using a column containing diatomaceous earth.

As shown in formula 15 below, the compound 6 of interest can be obtained by a method similar to that shown in formula 13 after the free amine 4 is obtained.

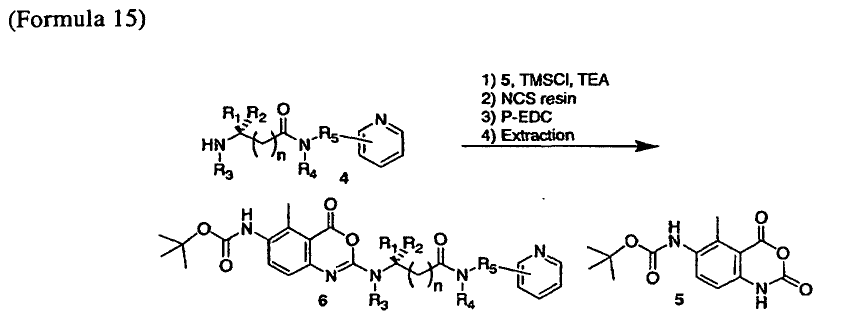

Formula 16 shows the process for synthesizing a compound in which R₆ is converted.

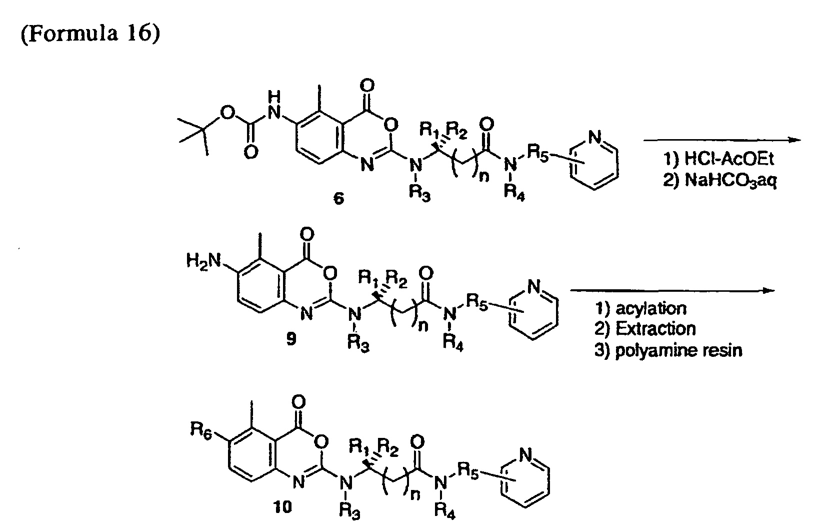

Boc in the compound 6 can be separated by acid treatment using HCl-containing ethyl acetate, and a free aniline body 9 can be obtained with the use of sodium bicarbonate water. Various acylation agents (e.g., acid chloride) are added to a solution of 9 in dichloromethane, and excess amounts of acylation agents are removed with the use of a polyamine resin after the parallel extraction using a column containing diatomaceous earth. Thus, a compound 10 of interest can be obtained.

Formula 17 shows the process for synthesizing a compound in which R₆ is converted into an amino acid derivative.

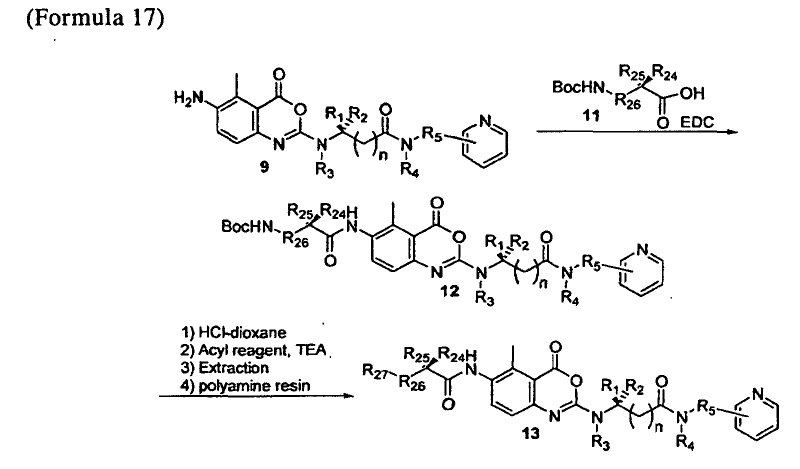

Boc-amino acid 11 is allowed to activate in the presence of EDC, followed by processing with the aniline body 9. Thus, a compound 12 can be obtained. Boc in the compound 12 can be separated by acid treatment using HCl-containing 1,4-dioxane, and various types of acylation agents (e.g., acid chloride) are added to the resulting hydrochloride, followed by parallel extraction using a column containing diatomaceous earth. Thereafter, excess amounts of acylation agents are removed with the use of a polyamine resin. Thus, a compound 13 of interest can be obtained.

Finally, formula 18 shows a process for synthesizing a compound in which R₆ is converted into a β-alanine derivative.

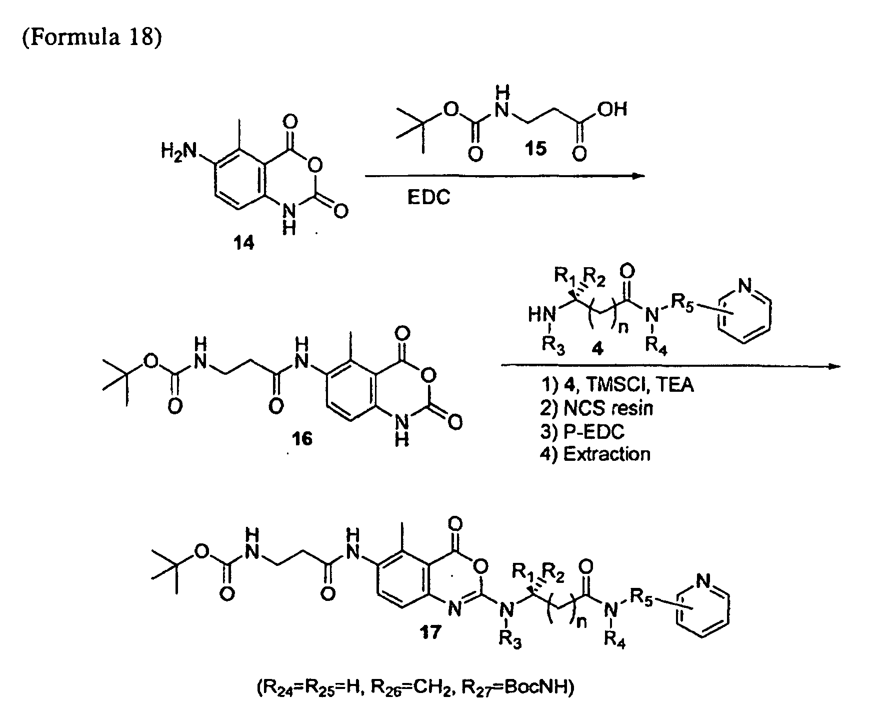

Boc-β-alanine 15 is allowed to activate in the presence of EDC, followed by processing with a known compound 14. Thus, a compound 16 can be obtained. Thereafter, a compound 17 of interest can be obtained in a manner similar to that shown in formula 13.

### Brief Description of the Drawings

Fig. 1 shows changes in scores of test compounds and those of the control group in the examination of antiviral activities (1) using mouse models to which ointments for HSV skin infections have been applied.
Fig. 2 shows changes in scores of the test for inspecting the combinational effect using mouse models with skin infections when a compound 18 is used as an ointment or oral preparation of acyclovir alone or in combination.

### Examples

Examples below contain detailed descriptions of the process for producing the compound represented by general formula 1. These detailed descriptions are within the scope of the present invention, and they illustrate the aforementioned general methods for synthesis that constitute a part of the present invention. These detailed descriptions are provided for illustrative purposes only, and are not intended to limit the scope of the present invention. Parts are by weight, and temperatures are of degrees centigrade, unless otherwise specified.

The following abbreviations are employed.
EtOAc - ethyl acetate
HCl - hydrochloric acid
DMSO - dimethyl sulfoxide
d₆ -DMSO - deuterated dimethyl sulfoxide
CDCl₃ - deuterated chloroform
CHCl₃ - chloroform
CD₃OD - deuterated methanol
EDC - 1-(3-dimethylaminopropyl)-3-ethylcarbodiimide hydrochloride
Et₂O - diethyl ether
MgSO₄ - magnesium sulfate
HOBt - N-hydroxybenzotriazole
H₂SO₄ - sulfuric acid
NaHCO₃ - sodium bicarbonate
KHSO₄ - potassium hydrogen sulfate
NMM - N-methylmorpholine
DMF - dimethylformamide
DMAP - 4-dimethylaminopyridine
CDI - carbonyldiimidazole
NaOH - sodium hydroxide
KOH - potassium hydroxide
LiOH - lithium hydroxide
Pd(OH)₂/C - palladium hydroxide on carbon
Pd/C - palladium on carbon
P-EDC - polymer-1-(3-dimethylaminopropyl)-3-ethylcarbodiimide
Boc - tert-butoxycarbonyl
TEA - triethylamine
TLC - thin layer chromatography
MeOH - methanol
KI - potassium iodide
DCM - dichloromethane
AcCI - acetic chloride
TMSCl - trimethylsilyl chloride
TFPA - tetrafluorophthalic anhydride
TFA - trifluoroacetec acid
THF - tetrahydrofuran
TBAF - tetrabutylammonium chloride
EtOH - ethanol

The present invention is hereafter described in more detail with reference to the following examples.

### Example 1

### Preparation of (5-methyl-2-{1-methyl-1-[methyl-(2-pyridin-2-yl-ethyl)-carbamoyl]-ethylamino}-4-oxo-4H-benzo[d][1,3]oxazin-6-yl)-carbamic acid tert-butyl ester (Compound No.18)

The reaction was carried out in accordance with the process shown in formula 13. EDC (10.06 g, 52.5 mmol), HOBt (7.10 g, 52.5 mmol), and NMM (5.76 ml, 52.5 mmol) were added in that order to a solution of Boc-aminoisobutyl acid (10.16 g, 50.0 mmol) in DCM (150 ml), the mixture was stirred at room temperature, 1-methylamino-2-(2-pyridyl)ethane (8.30 ml, 60.0 mmol) was added thereto 10 minutes later, and the resultant was stirred at room temperature for 15 hours. The reaction solution was washed with water (150 ml), dried over MgSO₄, and concentrated under reduced pressure. The resulting crude product was purified by silica gel column chromatography (eluent; CHCl₃:MeOH = 50:1). Thus, a colorless oily protected amide compound was obtained. This compound was dissolved in AcOEt (100 ml), 4N HCl-AcOEt (200 ml) was added thereto, and the mixture was stirred at room temperature for 2 hours. The solvent was removed under reduced pressure, an aqueous solution of 5N NaOH (100 ml) was added, and an oily suspended matter was extracted using AcOEt (150 ml × 2). The product was dried over MgSO₄ and concentrated under reduced pressure. Thus, free amine of interest was obtained (7.53 g, total yield 68.4%). TEA (18.9 ml, 135.9 mmol) and TMSC1 (8.7 ml, 68.0 mmol) were added in that order to a suspension of a known compound 5 (6.67 g, 22.7 mmol) in DCM (40 ml). After the mixture was stirred at room temperature for 30 minutes, the solution of free amine in DCM (20 ml) prepared above was added thereto, and the mixture was further stirred at room temperature for 60 minutes. The resultant was diluted with the addition of DCM (90 ml), washed with water (30 ml), and dried over magnesium sulfate. After the filtration, TEA (11.4 ml, 81.7 mmol) and AcCl (5.21 ml, 79.5 mmol) were added to the filtrate in that order, and the mixture was stirred at room temperature for 20 minutes. The resultant was washed with water (100 ml), dried over MgSO₄, and concentrated under reduced pressure. The resulting crude product was purified by silica gel column chromatography (eluent; AcOEt) to obtain the title compound (7.03 g, 62.8%) as a light yellow solid.
1H-NMR (300 MHz, CDCl₃): 1.45 (s, 9H), 1.59 (s, 6H), 2.63 (s, 3H), 2.98 (m, 2H), 3.06 (s, 3H), 3.77 (br, 2H), 6.29 (s, 1H), 7.12 (m, 3H), 7.57 (td, J = 7.5, 1.6 Hz, 1H), 7.83 (br, 1H), 8.50 (dd, J = 4.6, 1.6 Hz, 1H).
Mass spectrum (LCMS, pos: 496 (MH+)).
HPLC retention time (A): 5.56 minutes.

The following compounds were prepared in a manner similar to that described above, except that other suitable forms of Boc-amino acid 1 and pyridine ring-containing primary or secondary amine 2 were used instead of Boc-aminoisobutyl acid and 1-methylamino-2-(2-pyridyl)ethane.
(5-Methyl-2-{1-methyl-1-[methyl-(2-pyridin-3-yl-ethyl)-carbamoyl]-ethylamino}-4-oxo-4H-benzo[d][1,3]oxazin-6-yl)-carbamic acid tert-butyl ester (Compound No.19).
1H-NMR (300 MHz, CDCl₃): 1.50 (s, 9H), 1.68 (s, 6H), 2.63 (s, 3H), 2.98 (m, 2H), 3.06 (s, 3H), 3.77 (br, 2H), 5.92 (br, 1H), 6.34 (s, 1H), 7.11 (d, J = 8.7 Hz, 1H), 7.20 (m, 2H), 7.57 (m, 1H), 7.80 (br, 1H), 8.44 (m, 2H).
HPLC retention time (A): 5.24 minutes.
(5-Methyl-4-oxo-2-{2-phenyl-1S-[(pyridin-2-ylmethyl)-carbamoyl]-ethylamino}-4H-benzo[d][1,3]oxazin-6-yl)-carbamic acid tert-butyl ester (Compound No.20).
1H-NMR (300 MHz, CDCl₃): 1.44 (s, 9H), 2.56 (s, 3H), 3.16 (t, J = 6.9 Hz, 2H), 4.45 (m, 2H), 4.67 (br, 1H), 6.21 (s, 1H), 7.10 (m, 8H), 7.53 (td, J = 7.6, 2.0 Hz, 1H), 7.81 (br, 1H), 8.34 (d, J = 4.9 Hz, 1H).
Mass spectrum (FAB, pos: 530 (MH+)).
HPLC retention time (A): 6.66 minutes.
(5-Methyl-2-{1-[methyl-1S-(2-pyridin-2-yl-ethyl)-carbamoyl]-2-phenyl-ethylamino}-4-oxo-4H-benzo[d][1,3]oxazin-6-yl)-carbamic acid tert-butyl ester (Compound No.21).
1H-NMR (300 MHz, CDCl₃): 1.45 (s, 9H), 2.55 (s, 3H), 2.67, 2.80 (2s, 3H in combination), 3.02 (m, 4H), 3.27, 3.53 (2m, 2H in combination), 4.97 (br, 1H), 6.22 (s, 1H), 7.03 (m, 3H), 7.17 (m, 5H), 7.49 (m, 1H), 7.79 (br, 1H), 8.41 (2d, J = 4.9 Hz, 1H in combination).
Mass spectrum (FAB, pos: 558 (MH+)).
HPLC retention time (A): 7.49 minutes.
[5-Methyl-2-(methyl- {2-phenyl-1S-[(pyridin-2-ylmethyl)-carbamoyl]-ethyl}-amino)-4-oxo-4H-benzo[d][1,3]oxazin-6-yl]-carbamic acid tert-butyl ester (Compound No.22).
1H-NMR (300 MHz, CDCl₃): 1.52 (s, 9H), 2.61 (s, 3H), 3.06 (s, 3H), 3.13, 3.46 (2 m, 2H in combination), 4.54 (m, 2H), 5.39 (br, 1H), 6.28 (s, 1H), 7.17 (m, 8H), 7.57 (td, J = 7.5, 1.8 Hz, 1H), 7.87 (br, 1H), 8.38 (d, J = 4.5 Hz, 1H).
HPLC retention time (A): 7.04 minutes
{5-Methyl-2-[1S-(methyl-pyridin-2-ylmethyl-carbamoyl)-3-phenyl-propylamino]-4-oxo-4H-benzo[d][1,3]oxazin-6-yl}-carbamic acid tert-butyl ester (Compound No.23).
1H-NMR (300 MHz, CDCl₃): 1.57 (s, 9H), 2.13 (m, 2H), 2.58 (s, 3H), 2.70 (m, 2H), 3.05 (2s, 3H), 4.73 (m, 2H), 4.98 (m, 1H), 6.10 (br, 1H), 6.37 (br, 1H), 6.90 (m, 1H), 7.19 (m, 7H), 7.62 (2td, J = 7.5, 1.8 Hz, 1H), 7.80 (m, 1H), 8.53 (2d, J = 4.5 Hz, 1H).
HPLC retention time (A): 5.24 minutes.

### Example 2

### Another process for producing the Compound No.18

The reaction was carried out in accordance with the process shown in formula 14. P-EDC (1.80 mmol/g, 111 mg) prepared by a known technique was fractionated in a reaction vessel, a solution of Boc-aminoisobutyric acid in DCM (0.1M, 1.0 ml), a solution of HOBt in DCM (0.1M, 1.0 ml), and a solution of 1-methylamino-2-(2-pyridyl)ethane in DCM (0.2M, 0.5 ml) that were prepared in advance were added thereto in that order, and the mixture was shaken at room temperature for 15. hours. The reaction solution was filtered, a solution of TFPA in DCM (0.12M, 1.0 ml) was added thereto, and the mixture was shaken at room temperature for 2 hours. Thereafter, a polyamine resin (5.20 mmol/g, 38 mg) prepared by a known technique and Amberlist A-21 (5.00 mmol/g, 40 mg) were added, and the mixture was shaken at room temperature for an additional 15 hours. After the filtration, TFA (0.5 ml) was added to the filtrate, the resultant was shaken for 2.5 hours, and the solvent was then removed by distillation. MeOH (2.0 ml) was added to the residue to prepare a solution, and the solvent was removed by distillation again. DCM (1.0 ml) was added to the residue, Amberlist A-21(100 mg) was added thereto, and the resultant was shaken at room temperature for 6 hours, followed by filtration. A solution of a known compound 7 in DCM (0.05M, 1.0 ml) was added to the filtrate, and the resultant was shaken at room temperature for 48 hours. A solution of TFPA in DCM (0.15M, 1.0 ml) was added to the reaction solution, the resultant was shaken at room temperature for 3 hours, a polyamine resin (77 mg) was added thereto, and the resultant was then shaken at room temperature for 2 hours. After the filtration, a polyamine resin (77 mg) was added, the resultant was shaken at room temperature for 2 hours, the resultant was filtered, and the solvent was removed by distillation under reduced pressure. THF (2.0 ml) was added to the residue to prepare a solution, a solution of TBAF (1.0M, 0.06 ml) in THF was added thereto, and the resultant was shaken at room temperature for 3 hours. After the solvent was removed by distillation under reduced pressure, DCM (2.0 ml) was added to prepare a solution, and TEA (0.017 ml) and AcCl (0.008 ml) were added, followed by shaking for 30 minutes. The reaction solution was allowed to pass through a column containing diatomaceous earth (0.564 g filled) that had previously been allowed to retain water (0.5 ml), and DCM (1.0 ml) was further allowed to pass therethrough. After the concentration under reduced pressure, the resulting crude product was purified by silica gel column chromatography (eluent; DCM:EtOH = 19:1 to 10:1). Thus, the title compound (2.3 mg, total yield 9.3%) was obtained as a light yellow solid. The following compounds were prepared in a similar manner, except that other suitable forms of Boc-amino acid 1 and pyridine ring-containing primary or secondary amine 2 were used instead of Boc-aminoisobutyric acid and 1-methylamino-2-(2-pyridyl)ethane.
(5-Methyl-2-{2-methyl-1S-[(pyridin-3-ylmethyl)-carbamoyl]-propylamino}-4-oxo-4H-benzo[d][1,3]oxazin-6-yl)-carbamic acid tert-butyl ester (Compound No.24). HPLC retention time (A): 5.30 minutes.
(5-Methyl-2- {2-methyl-1S-[(pyridin-4-ylmethyl)-carbamoyl]-propylamino}-4-oxo-4H-benzo[d][1,3]oxazin-6-yl)-carbamic acid tert-butyl ester (Compound No.25). HPLC retention time (A): 5.18 minutes.
{5-Methyl-2-[2-methyl-1S-(2-pyridin-2-ylethylcarbamoyl)-propylamino]-4-oxo-4H-benzo[d][1,3]oxazin-b-yl}-carbamic acid tert-butyl ester (Compound No.26). HPLC retention time (A): 6.02 minutes.
(2- {2-Cyclohexyl-1S-[(pyridin-3-ylmethyl)-carbamoyl]-ethylamino}-5-methyl-4-oxo-4H-benzo[d][1,3]oxazin-6-yl)-carbamic acid tert-butyl ester (Compound No.27). HPLC retention time (A): 7.22 minutes.
(2-{2-Cyclohexyl-1S-[(pyridin-4-ylmethyl)-carbamoyl]-ethylamino}-5-methyl-4-oxo-4H-benzo[d][1,3]oxazin-6-yl)-carbamic acid tert-butyl ester (Compound No.28). HPLC retention time (A): 7.17 minutes.
{2-[2-Cyclohexyl-1S-(2-pyridin-2-ylethylcarbamoyl)-ethylamino]-5-methyl-4-oxo-4H-benzo[d][1,3]oxazin-6-yl}-carbamic acid tert-butyl ester (Compound No.29). HPLC retention time (A): 7.42 minutes.
(5-Methyl-4-oxo-2-{2-phenyl-1S-[(pyridin-3-ylmethyl)-carbamoyl]-ethylamino}-4H-benzo[d][1,3]oxazin-6-yl)-carbamic acid tert-butyl ester (Compound No.30). HPLC retention time (A): 6.45 minutes.
(5-Methyl-4-oxo-2-{2-phenyl-1S-[(pyridin-4-ylmethyl)-carbamoyl]-ethylamino}-4H-benzo[d][1,3]oxazin-6-yl)-carbamic acid tert-butyl ester (Compound No.31). HPLC retention time (A): 5.86 minutes.
(2- {2,2-dimethyl-1S-[(pyridin-2-ylmethyl)-carbamoyl]-propylamino}-5-methyl-4-oxo-4H-benzo[d][1,3]oxazin-6-yl)-carbamic acid tert-butyl ester (Compound No.32). HPLC retention time (A): 6.44 minutes.
(2-{2,2-dimethyl-1S-[(pyridin-3-ylmethyl)-carbamoyl]-propylamino}-5-methyl-4-oxo-4H-benzo[d][1,3]oxazin-6-yl)-carbamic acid tert-butyl ester (Compound No.33). HPLC retention time (A): 6.17 minutes.
(5-Methyl-2-{1-methyl-1-[(pyridin-4-ylmethyl)-carbamoyl]-ethylamino}-4-oxo-4H-benzo[d][1,3]oxazin-6-yl)-carbamic acid tert-butyl ester (Compound No.34). HPLC retention time (A): 4.90 minutes.
{5-Methyl-2-[1-methyl-1-(2-pyridin-2-ylethylcarbamoyl)-ethylamino]-4-oxo-4H-benzo[d][1,3]oxazin-6-yl}-carbamic acid tert-butyl ester (Compound No.35). HPLC retention time (A): 5.52 minutes.
(5-Methyl-2-{3-methyl-1S-[(pyridin-2-ylmethyl)-carbamoyl]-butylamino}-4-oxo-4H-benzo[d][1,3]oxazin-6-yl)-carbamic acid tert-butyl ester (Compound No.36). HPLC retention time (A): 6.54 minutes.
(5-Methyl-2- {3-methyl-1S-[(pyridin-3-ylmethyl)-carbamoyl]-butylamino}-4-oxo-4H-benzo[d][1,3]oxazin-6-yl)-carbamic acid tert-butyl ester (Compound No.37). HPLC retention time (A): 6.35 minutes.
(5-Methyl-2- {3-methyl-1S-[(pyridin-4-ylmethyl)-carbamoyl]-butylamino}-4-oxo-4H-benzo[d][1,3]oxazin-6-yl)-carbamic acid tert-butyl ester (Compound No.38). HPLC retention time (A): 6.28 minutes.
(5-Methyl-4-oxo-2- {3-phenyl-1S-[(pyridin-2-ylmethyl)-carbamoyl]-propylamino}-4H-benzo[d][1,3]oxazin-6-yl)-carbamic acid tert-butyl ester (Compound No.39). HPLC retention time (A): 7.00 minutes.
(5-Methyl-4-oxo-2-{3-phenyl-1S-[(pyridin-3-ylmethyl)-carbamoyl]-propylamino}-4H-benzo[d][1,3]oxazin-6-yl)-carbamic acid tert-butyl ester (Compound No.40). HPLC retention time (A): 6.80 minutes.
(5-Methyl-4-oxo-2-{3-phenyl-1S-[(pyridin-4-ylmethyl)-carbamoyl]-propylamino}-4H-benzo[d][1,3]oxazin-6-yl)-carbamic acid tert-butyl ester (Compound No.41). HPLC retention time (A): 6.76 minutes.
{5-Methyl-4-oxo-2-[3-phenyl-1S-(2-pyridin-2-yl-ethylcarbamoyl)-propylamino]-4H-benzo[d][1,3]oxazin-6-yl}-carbamic acid tert-butyl ester (Compound No.42). HPLC retention time (A): 7.00 minutes.
(5-Methyl-2-{1S-[methyl-(2-pyridin-2-yl-ethyl)-carbamoyl]-ethylamino}-4-oxo-4H-benzo[d][1,3]oxazin-6-yl)-carbamic acid tert-butyl ester (Compound No.43). HPLC retention time (A): 5.59 minutes.
(2- {2,2-dimethyl-1S-[methyl-(2-pyridin-2-yl-ethyl)-carbamoyl)-propylamino]-5-methyl-4-oxo-4H-benzo[d][1,3]oxazin-6-yl)-carbamic acid tert-butyl ester (Compound No.44). HPLC retention time (A): 6.97 minutes.
(5-Methyl-2- {2-methyl-1S-[methyl-(2-pyridin-2-yl-ethyl)-carbamoyl]-propylamino}-4-oxo-4H-benzo[d][1,3]oxazin-6-yl)-carbamic acid tert-butyl ester (Compound No.45). HPLC retention time (A): 6.49 minutes.
(5-Methyl-2- {3-methyl-1S-[methyl-(2-pyridin-2-yl-ethyl)-carbamoyl]-butylamino}-4-oxo-4H-benzo[d][1,3]oxazin-6-yl)-carbamic acid tert-butyl ester (Compound No.46). HPLC retention time (A): 6.94 minutes.
(2-{2-cyclohexyl-1S-[methyl-(2-pyridin-2-yl-ethyl)-carbamoyl]-ethylamino}-5-methyl-4-oxo-4H-benzo[d][1,3]oxazin-6-yl)-carbamic acid tert-butyl ester (Compound No.47). HPLC retention time (A): 7.83 minutes.
(5-Methyl-2- {1S-[methyl-(2-pyridin-2-yl-ethyl)-carbamoyl]-3-phenyl-propylamino}-4-oxo-4H-benzo[d][1,3]oxazin-6-yl)-carbamic acid tert-butyl ester (Compound No.48). HPLC retention time (A): 7.17 minutes.
(5-Methyl-2-{1S-[methyl-(2-pyridin-4-yl-ethyl)-carbamoyl]-2-phenyl-ethylamino}-4-oxo-4H-benzo[d][1,3]oxazin-6-yl)-carbamic acid tert-butyl ester (Compound No.49). HPLC retention time (A): 6.90 minutes.
(5-Methyl-2-{1S-[methyl-(2-pyridin-4-yl-ethyl)-carbamoyl]-ethylamino}-4-oxo-4H-benzo[d][1,3]oxazin-6-yl)-carbamic acid tert-butyl ester (Compound No.50). HPLC retention time (A): 5.45 minutes.
(5-Methyl-2-{1-methyl-1-[methyl-(2-pyridin-4-yl-ethyl)-carbamoyl]-ethylamino}-4-oxo-4H-benzo[d][1,3]oxazin-6-yl)-carbamic acid tert-butyl ester (Compound No.51). HPLC retention time (A): 5.40 minutes.
(2-{2,2-Dimethyl-1S-[methyl-(2-pyridin-4-yl-ethyl)-carbamoyl]-propylamino}-5-methyl-4-oxo-4H-benzo[d][1,3]oxazin-6-yl)-carbamic acid tert-butyl ester (Compound No.52). HPLC retention time (A): 6.94 minutes.
(5-Methyl-2-{2-methyl-1S-[methyl-(2-pyridin-4-yl-ethyl)-carbamoyl]-propylamino}-4-oxo-4H-benzo[d][1,3]oxazin-6-yl)-carbamic acid tert-butyl ester (Compound No.53). HPLC retention time (A): 6.43 minutes.
(5-Methyl-2-{3-methyl-1S-[methyl-(2-pyridin-4-yl-ethyl)-carbamoyl]-butylamino}-4-oxo-4H-benzo[d][1,3]oxazin-6-yl)-carbamic acid tert-butyl ester (Compound No.54). HPLC retention time (A): 6.88 minutes.
(2-{2-Cyclohexyl-1S-[methyl-(2-pyridin-4-yl-ethyl)-carbamoyl]-ethylamino}-5-methyl-4-oxo-4H-benzo[d][1,3]oxazin-6-yl)-carbamic acid tert-butyl ester (Compound No.55). HPLC retention time (A): 7.82 minutes.
(5-Methyl-2-{1S-[methyl-(2-pyridin-4-yl-ethyl)-carbamoyl]-3-phenyl-propylamino}-4-oxo-4H-benzo[d][1,3]oxazin-6-yl)-carbamic acid tert-butyl ester (Compound No.56). HPLC retention time (A): 7.15 minutes.
{5-Methyl-2-[1S-(methyl-pyridin-2-ylmethyl-carbamoyl)-2-phenyl-ethylamino]-4-oxo-4H-benzo[d][1,3]oxazin-6-yl}-carbamic acid tert-butyl ester (Compound No.57). HPLC retention time (A): 7.12 minutes.
{5-Methyl-2-[1S-(methyl-pyridin-2-ylmethyl-carbamoyl)-ethylamino]-4-oxo-4H-benzo[d][1,3]oxazin-6-yl}-carbamic acid tert-butyl ester (Compound No.58). HPLC retention time (A): 5.69 minutes.
{5-Methyl-2-[1-methyl-1-(methyl-pyridin-2-ylmethyl-carbamoyl)-ethylamino]-4-oxo-4H-benzo[d][1,3]oxazin-6-yl}-carbamic acid tert-butyl ester (Compound No.59). HPLC retention time (A): 5.50 minutes.
{2-[2,2-Dimethyl-1S-(methyl-pyridin-2-ylmethyl-carbamoyl)-propylamino]-5-methyl-4-oxo-4H-benzo[d][1,3]oxazin-6-yl}-carbamic acid tert-butyl ester (Compound No.60). HPLC retention time (A): 7.11 minutes.
{5-Methyl-2-[2-methyl-1S-(methyl-pyridin-2-ylmethyl-carbamoyl)-propylamino]-4-oxo-4H-benzo[d][1,3]oxazin-6-yl}-carbamic acid tert-butyl ester (Compound No.61). HPLC retention time (A): 6.64 minutes.
{5-Methyl-2-[3-methyl-1S-(methyl-pyridin-2-ylmethyl-carbamoyl)-butylamino]-4-oxo-4H-benzo[d][1,3]oxazin-6-yl}-carbamic acid tert-butyl ester (Compound No.62). HPLC retention time (A): 7.12 minutes.
{2-[2-Cyclohexyl-1S-(methyl-pyridin-2-ylmethyl-carbamoyl)-ethylamino]-5-methyl-4-oxo-4H-benzo[d][1,3]oxazin-6-yl}-carbamic acid tert-butyl ester (Compound No.63). HPLC retention time (A): 8.13 minutes.
(5-Methyl-2-{1S-[methyl-(2-pyridin-3-yl-ethyl)-carbamoyl]-2-phenyl-ethylamino}-4-oxo-4H-benzo[d][1,3]oxazin-6-yl)-carbamic acid tert-butyl ester (Compound No.64). HPLC retention time (A): 7.16 minutes.
(5-Methyl-2-{1S-[methyl-(2-pyridin-3-yl-ethyl)-carbamoyl]-ethylamino}-4-oxo-4H-benzo[d][1,3]oxazin-6-yl)-carbamic acid tert-butyl ester (Compound No.65). HPLC retention time (A): 5.84 minutes.
(2-{2,2-Dimethyl-1S-[methyl-(2-pyridin-3-yl-ethyl)-carbamoyl]-propylamino}-5-methyl-4-oxo-4H-benzo[d][1,3]oxazin-6-yl)-carbamic acid tert-butyl ester (Compound No.66). HPLC retention time (A): 7.14 minutes.
(5-Methyl-2-{2-methyl-1S-[methyl-(2-pyridin-3-yl-ethyl)-carbamoyl]-propylamino}-4-oxo-4H-benzo[d][1,3]oxazin-6-yl)-carbamic acid tert-butyl ester (Compound No.67). HPLC retention time (A): 6.68 minutes.
(5-Methyl-2-{3-methyl-1S-[methyl-(2-pyridin-3-yl-ethyl)-carbamoyl]-butylamino}-4-oxo-4H-benzo[d][1,3]oxazin-6-yl)-carbamic acid tert-butyl ester (Compound No.68). HPLC retention time (A): 7.17 minutes.
(2-{2-Cyclohexyl-1S-[methyl-(2-pyridin-3-yl-ethyl)-carbamoyl]-ethylamino}-5-methyl-4-oxo-4H-benzo[d][1,3]oxazin-6-yl)-carbamic acid tert-butyl ester (Compound No.69). HPLC retention time (A): 8.10 minutes.
(5-Methyl-2-{1S-[methyl-(2-pyridin-3-yl-ethyl)-carbamoyl]-3-phenyl-propylamino}-4-oxo-4H-benzo[d][1,3]oxazin-6-yl)-carbamic acid tert-butyl ester (Compound No.70). HPLC retention time (A): 7.76 minutes.
[5-Methyl-2-(methyl-{1S-[(pyridin-2-ylmethyl)-carbamoyl]-ethyl}-amino)-4-oxo-4H-benzo[d][1,3]oxazin-6-yl]-carbamic acid tert-butyl ester (Compound No.71). HPLC retention time (A): 6.39 minutes.
[5-Methyl-2-(methyl-{1S-[(pyridin-3-ylmethyl)-carbamoyl]-ethyl}-amino)-4-oxo-4H-benzo[d][1,3]oxazin-6-yl]-carbamic acid tert-butyl ester (Compound No.72). HPLC retention time (A): 5.72 minutes.
[5-Methyl-2-(methyl-{1S-[(pyridin-4-ylmethyl)-carbamoyl]-ethyl}-amino)-4-oxo-4H-benzo[d][1,3]oxazin-6-yl]-carbamic acid tert-butyl ester (Compound No.73). HPLC retention time (A): 5.67 minutes.
(5-Methyl-2- {methyl-[1S-(2-pyridin-2-ylethylcarbamoyl)-ethyl]-amino}-4-oxo-4H-benzo[d][1,3]oxazin-6-yl)-carbamic acid tert-butyl ester (Compound No.74). HPLC retention time (A): 6.12 minutes.
(5-Methyl-2-{methyl-[1S-(methyl-pyridin-2-ylmethyl-carbamoyl)-ethyl]-amino}-4-oxo-4H-benzo[d][1,3]oxazin-6-yl)-carbamic acid tert-butyl ester (Compound No.75). Mass spectrum (FAB, pos: 482 (MH+)). HPLC retention time (A): 6.38 minutes.
[5-Methyl-2-(methyl-{1S-[methyl-(2-pyridin-2-yl-ethyl)-carbamoyl]-ethyl}-amino)-4-oxo-4H-benzo[d][1,3]oxazin-6-yl]-carbamic acid tert-butyl ester (Compound No.76). HPLC retention time (A): 6.54 minutes.
[5-Methyl-2-(methyl-{1S-[methyl-(2-pyridin-3-yl-ethyl)-carbamoyl]-ethyl}-amino)-4-oxo-4H-benzo[d][1,3]oxazin-6-yl]-carbamic acid tert-butyl ester (Compound No.77). HPLC retention time (A): 6.42 minutes.
[5-Methyl-2-(methyl-{1S-[methyl-(2-pyridin-4-yl-ethyl)-carbamoyl]-ethyl}-amino)-4-oxo-4H-benzo[d][1,3]oxazin-6-yl]-carbamic acid tert-butyl ester (Compound No.78). HPLC retention time (A): 6.34 minutes.
[5-Methyl-2-(methyl-{2-phenyl-1S-[(pyridin-3-ylmethyl)-carbamoyl]-ethyl}-amino)-4-oxo-4H-benzo[d][1,3]oxazin-6-yl]-carbamic acid tert-butyl ester (Compound No.79). HPLC retention time (A): 7.21 minutes.
[5-Methyl-2-(methyl-{2-phenyl-1S-[(pyridin-4-ylmethyl)-carbamoyl]-ethyl}-amino)-4-oxo-4H-benzo[d][1,3]oxazin-6-yl]-carbamic acid tert-butyl ester (Compound No.80). HPLC retention time (A): 7.18 minutes.
(5-Methyl-2-{methyl-[2-phenyl-1S-(2-pyridin-2-yl-ethylcarbamoyl)-ethyl]-amino}-4-oxo-4H-benzo[d][1,3]oxazin-6-yl)-carbamic acid tert-butyl ester (Compound No.81). HPLC retention time (A): 7.42 minutes.
(5-Methyl-2-{methyl-[1S-(methyl-pyridin-2-ylmethyl-carbamoyl)-2-phenyl-ethyl]-amino}-4-oxo-4H-benzo[d][1,3]oxazin-6-yl)-carbamic acid tert-butyl ester (Compound No.82). Mass spectrum (FAB, pos: 558 (MH+)). HPLC retention time (A): 7.56 minutes.
[5-Methyl-2-(methyl-{1S-[methyl-(2-pyridin-2-yl-ethyl)carbamoyl]-2-phenyl-ethyl}-amino)-4-oxo-4H-benzo[d][1,3]oxazin-6-yl]-carbamic acid tert-butyl ester (Compound No.83). HPLC retention time (A): 7.75 minutes.
[5-Methyl-2-(methyl-{1S-[methyl-(2-pyridin-3-yl-ethyl)-carbamoyl]-2-phenyl-ethyl}-amino)-4-oxo-4H-benzo[d][1,3]oxazin-6-yl]-carbamic acid tert-butyl ester (Compound No.84). HPLC retention time (A): 7.61 minutes.
[5-Methyl-2-(methyl-{1S-[methyl-(2-pyridin-4-yl-ethyl)-carbamoyl]-2-phenyl-ethyl}-amino)-4-oxo-4H-benzo[d][1,3]oxazin-6-yl]-carbamic acid tert-butyl ester (Compound No.85). HPLC retention time (A): 7.56 minutes.
(5-Methyl-4-oxo-2-{2S-[(pyridin-2-ylmethyl)-carbamoyl]-pyrrolidin-1-yl}-4H-benzo[d][1,3]oxazin-6-yl)-carbamic acid tert-butyl ester (Compound No.86). HPLC retention time (A): 5.69 minutes.
(5-Methyl-4-oxo-2-{2S-[(pyridin-3-ylmethyl)-carbamoyl]-pyrrolidin-1-yl}-4H-benzo[d][1,3]oxazin-6-yl)-carbamic acid tert-butyl ester (Compound No.87). HPLC retention time (A): 5.39 minutes.
(5-Methyl-4-oxo-2-{2S-[(pyridin-4-ylmethyl)-carbamoyl]-pyrrolidin-1-yl}-4H-benzo[d][1,3]oxazin-6-yl)-carbamic acid tert-butyl ester (Compound No.88). HPLC retention time (A): 5.23 minutes.
{5-Methyl-4-oxo-2-[2S-(2-pyridin-2-ylethylcarbamoyl)-pyrrolidin-1-yl]-4H-benzo[d][1,3]oxazin-6-yl}-carbamic acid tert-butyl ester (Compound No.89). HPLC retention time (A): 5.76 minutes.
{5-Methyl-2-[2S-(methyl-pyridin-2-ylmethyl-carbamoyl)-pyrrolidin-1-yl]-4-oxo-4H-benzo[d][1,3]oxazin-6-yl}-carbamic acid tert-butyl ester (Compound No.90). Mass spectrum (FAB, pos: 494 (MH+)). HPLC retention time (A): 5.90 minutes.
(5-Methyl-2-{2S-[methyl-(2-pyridin-2-yl-ethyl)-carbamoyl]-pyrrolidin-1-yl}-4-oxo-4H-benzo[d][1,3]oxazin-6-yl)-carbamic acid tert-butyl ester (Compound No.91). HPLC retention time (A): 6.16 minutes.
(5-Methyl-2-{2S-[methyl-(2-pyridin-3-yl-ethyl)-carbamoyl]-pyrrolidin-1-yl}-4-oxo-4H-benzo[d][1,3]oxazin-6-yl)-carbamic acid tert-butyl ester (Compound No.92). HPLC retention time (A): 5.99 minutes.
(5-Methyl-2-{2S-[methyl-(2-pyridin-4-yl-ethyl)-carbamoyl]-pyrrolidin-1-yl}-4-oxo-4H-benzo[d][1,3]oxazin-6-yl)-carbamic acid tert-butyl ester (Compound No.93). HPLC retention time (A): 5.88 minutes.
(5-Methyl-4-oxo-2-{3S-[(pyridin-2-ylmethyl)-carbamoyl]-3,4-dihydro-1H-isoquinolin-2-yl}-4H-benzo[d][1,3]oxazin-6-yl)-carbamic acid tert-butyl ester (Compound No.94). HPLC retention time (A): 7.16 minutes.
(5-Methyl-4-oxo-2-{3S-[(pyridin-3-ylmethyl)-carbamoyl]-3,4-dihydro-1H-isoquinolin-2-yl}-4H-benzo[d][1,3]oxazin-6-yl)-carbamic acid tert-butyl ester (Compound No.95). HPLC retention time (A): 6.94 minutes.
(5-Methyl-4-oxo-2-{3S-[(pyridin-4-ylmethyl)-carbamoyl]-3,4-dihydro-1H-isoquinolin-2-yl}-4H-benzo[d][1,3]oxazin-6-yl)-carbamic acid tert-butyl ester (Compound No.96). HPLC retention time (A): 6.88 minutes.
{5-Methyl-4-oxo-2-[3S-(2-pyridin-2-yl-ethylcarbamoyl)-3,4-dihydro-1H-isoquinolin-2-yl]-4H-benzo[d][1,3]oxazin-6-yl}-carbamic acid tert-butyl ester (Compound No.97). HPLC retention time (A): 7.28 minutes.
{5-Methyl-2-[3S-(methyl-pyridin-2-yl-methylcarbamoyl)-3,4-dihydro-1H-isoquinolin-2-yl]-4-oxo-4H-benzo[d][1,3]oxazin-6-yl}-carbamic acid tert-butyl ester (Compound No.98). Mass spectrum (FAB, pos: 556 (MH+)). HPLC retention time (A): 7.38 minutes.
(5-Methyl-2-{3S-[methyl-(2-pyridin-2-yl-ethyl)-carbamoyl]-3,4-dihydro-1H-isoquinolin-2-yl}-4-oxo-4H-benzo[d][1,3]oxazin-6-yl)-carbamic acid tert-butyl ester (Compound No.99). HPLC retention time (A): 7.68 minutes.
(5-Methyl-2-{3S-[methyl-(2-pyridin-3-yl-ethyl)-carbamoyl]-3,4-dihydro-1H-isoquinolin-2-yl}-4-oxo-4H-benzo[d][1,3]oxazin-6-yl)-carbamic acid tert-butyl ester (Compound No. 100). HPLC retention time (A): 7.51 minutes.
(5-Methyl-2-{3S-[methyl-(2-pyridin-4-yl-ethyl)-carbamoyl]-3,4-dihydro-1H-isoquinolin-2-yl}-4-oxo-4H-benzo[d][1,3]oxazin-6-yl)-carbamic acid tert-butyl ester (Compound No.101). HPLC retention time (A): 7.50 minutes.
(5-Methyl-4-oxo-2-{2S-[(pyridin-2-ylmethyl)-carbamoyl]-piperidin-1-yl}-4H-benzo[d][1,3]oxazin-6-yl)-carbamic acid tert-butyl ester (Compound No.102). Mass spectrum (FAB, pos: 494 (MH+)). HPLC retention time (A): 5.77 minutes.
(5-Methyl-4-oxo-2-{2S-[(pyridin-3-ylmethyl)-carbamoyl]-piperidin-1-yl}-4H-benzo[d][1,3]oxazin-6-yl)-carbamic acid tert-butyl ester (Compound No.103). Mass spectrum (FAB, pos: 494 (MH+)). HPLC retention time (A): 5.46 minutes.
(5-Methyl-4-oxo-2-{2S-[(pyridin-4-ylmethyl)-carbamoyl]-piperidin-1-yl}-4H-benzo[d][1,3]oxazin-6-yl)-carbamic acid tert-butyl ester (Compound No.104). Mass spectrum (FAB, pos: 494 (MH+)). HPLC retention time (A): 5.31 minutes.
{5-Methyl-4-oxo-2-[2S-(2-pyridin-2-ylethylcarbamoyl)-piperidin-1-yl]-4H-benzo[d][1,3]oxazin-6-yl}-carbamic acid tert-butyl ester (Compound No.105). Mass spectrum (FAB, pos: 508 (MH+)). HPLC retention time (A): 5.88 minutes.
(5-Methyl-2-{2S-[methyl-(2-pyridin-2-yl-ethyl)-carbamoyl]-piperidin-1-yl}-4-oxo-4H-benzo[d][1,3]oxazin-6-yl)-carbamic acid tert-butyl ester (Compound No.106). Mass spectrum (FAB, pos: 522 (MH+)). HPLC retention time (A): 6.26 minutes.
(5-Methyl-2-{2S-[methyl-(2-pyridin-3-yl-ethyl)-carbamoyl]-piperidin-1-yl}-4-oxo-4H-benzo[d][1,3]oxazin-6-yl)-carbamic acid tert-butyl ester (Compound No.107). Mass spectrum (FAB, pos: 522 (MH+)). HPLC retention time (A): 6.08 minutes.
(5-Methyl-2-{2S-[methyl-(2-pyridin-4-yl-ethyl)-carbamoyl]-piperidin-1-yl}-4-oxo-4H-benzo[d][1,3]oxazin-6-yl)-carbamic acid tert-butyl ester (Compound No.108). Mass spectrum (FAB, pos: 522 (MH+)). HPLC retention time (A): 6.03 minutes.
(2-{4R-Benzyloxy-2S-[(pyridin-2-ylmethyl)-carbamoyl]-pyrrolidin-1-yl}-5-methyl-4-oxo-4H-benzo[d][1,3]oxazin-6-yl)-carbamic acid tert-butyl ester (Compound No.109). HPLC retention time (A): 7.28 minutes.
(2-{4R-Benzyloxy-2S-[(pyridin-3-ylmethyl)-carbamoyl]-pyrrolidin-1-yl}-5-methyl-4-oxo-4H-benzo[d][1,3]oxazin-6-yl)-carbamic acid tert-butyl ester (Compound No.110). HPLC retention time (A): 7.10 minutes.
(2-{4R-Benzyloxy-2S-[(pyridin-4-ylmethyl)-carbamoyl]-pyrrolidin-1-yl}-5-methyl-4-oxo-4H-benzo[d][1,3]oxazin-6-yl)-carbamic acid tert-butyl ester (Compound No.111), HPLC retention time (A): 7.05 minutes.
{2-[4R-Benzyloxy-2S-(2-pyridin-2-ylethylcarbamoyl)-pyrrolidin-1-yl]-5-methyl-4-oxo-4H-benzo[d][1,3]oxazin-6-yl}-carbamic acid tert-butyl ester (Compound No.112). HPLC retention time (A): 7.32 minutes.
{2-[4R-Benzyloxy-2S-(methyl-pyridin-2-ylmethyl-carbamoyl)-pyrrolidin-1-yl]-5-methyl-4-oxo-4H-benzo[d][1,3]oxazin-6-yl}-carbamic acid tert-butyl ester (Compound No.113). Mass spectrum (FAB, pos: 600 (MH+)). HPLC retention time (A): 7.35 minutes.
(2-{4R-Benzyloxy-2S-[methyl-(2-pyridin-2-yl-ethyl)-carbamoyl]-pyrrolidin-1-yl}-5-methyl-4-oxo-4H-benzo[d][1,3]oxazin-6-yl)-carbamic acid tert-butyl ester (Compound No.114). HPLC retention time (A): 7.66 minutes.
(2-{4R-Benzyloxy-2S-[methyl-(2-pyridin-3-yl-ethyl)-carbamoyl]-pyrrolidin-1-yl}-5-methyl-4-oxo-4H-benzo[d][1,3]oxazin-6-yl)-carbamic acid tert-butyl ester (Compound No.115). HPLC retention time (A): 7.52 minutes.
(2-{4R-Benzyloxy-2S-[methyl-(2-pyridin-4-yl-ethyl)-carbamoyl]-pyrrolidin-1-yl}-5-methyl-4-oxo-4H-benzo[d][1,3]oxazin-6-yl)-carbamic acid tert-butyl ester (Compound No.116). HPLC retention time (A): 7.48 minutes.
{5-Methyl-2-[1-methyl-1-(methyl-pyridin-3-ylmethyl-carbamoyl)-ethylamino]-4-oxo-4H-benzo[d][1,3]oxazin-6-yl}-carbamic acid tert-butyl ester (Compound No.117). Mass spectrum (LCMS, pos: 482 (MH+)). HPLC retention time (A): 4.96 minutes.
{5-Methyl-2-[1-(methyl-pyridin-3-ylmethyl-carbamoyl)-cyclopropylamino]-4-oxo-4H-benzo[d][1,3]oxazin-6-yl}-carbamic acid tert-butyl ester (Compound No.118). Mass spectrum (LCMS, pos: 480 (MH+)). HPLC retention time (A): 3.93 minutes.
{5-Methyl-2-[1-(methyl-pyridin-3-ylmethyl-carbamoyl)-cyclopentylamino]-4-oxo-4H-benzo[d][1,3]oxazin-6-yl)-carbamic acid tert-butyl ester (Compound No.119). Mass spectrum (LCMS, pos: 508 (MH+)). HPLC retention time (A): 5.77 minutes.
{5-Methyl-2-[1-(methyl-pyridin-3-ylmethyl-carbamoyl)-cyclohexylamino]-4-oxo-4H-benzo[d][1,3]oxazin-6-yl)-carbamic acid tert-butyl ester (Compound No.120). Mass spectrum (LCMS, pos: 522 (MH+)). HPLC retention time (A): 6.36 minutes.
{5-Methyl-2-[1-(methyl-pyridin-4-ylmethyl-carbamoyl)-cyclopentylamino]-4-oxo-4H-benzo[d][1,3]oxazin-6-yl}-carbamic acid tert-butyl ester (Compound No.121). Mass spectrum (LCMS, pos: 508 (MH+)). HPLC retention time (A): 5.74 minutes.
(5-Methyl-2-{1-[methyl-(2-pyridin-2-yl-ethyl)-carbamoyl]-cyclopropylamino}-4-oxo-4H-benzo[d][1,3]oxazin-6-yl)-carbamic acid tert-butyl ester (Compound No.122). Mass spectrum (LCMS, pos: 494 (MH+)). HPLC retention time (A): 4.94 minutes.
(5-Methyl-2-{1-[methyl-(2-pyridin-2-yl-ethyl)-carbamoyl]-cyclopentylamino}-4-oxo-4H-benzo[d][1,3]oxazin-6-yl)-carbamic acid tert-butyl ester (Compound No.123). Mass spectrum (LCMS, pos: 522 (MH+)). HPLC retention time (A): 6.21 minutes.
(5-Methyl-2-{1-[methyl-(2-pyridin-2-yl-ethyl)-carbamoyl]-cyclohexylamino}-4-oxo-4H-benzo[d][1,3]oxazin-6-yl)-carbamic acid tert-butyl ester (Compound No.124). Mass spectrum (LCMS, pos: 536 (MH+)). HPLC retention time (A): 6.64 minutes.
(5-Methyl-2-{1-[methyl-(2-pyridin-3-yl-ethyl)-carbamoyl]-cyclopropylamino}-4-oxo-4H-benzo[d][1,3]oxazin-6-yl)-carbamic acid tert-butyl ester (Compound No.125). Mass spectrum (LCMS, pos: 494 (MH+)). HPLC retention time (A): 4.45 minutes.
(5-Methyl-2-{1-[methyl-(2-pyridin-3-yl-ethyl)-carbamoyl]-cyclopentylamino}-4-oxo-4H-benzo[d][1,3]oxazin-6-yl)-carbamic acid tert-butyl ester (Compound No.126). Mass spectrum (LCMS, pos: 522 (MH+)). HPLC retention time (A): 6.03 minutes.
(5-Methyl-2-{1-[methyl-(2-pyridin-4-yl-ethyl)-carbamoyl]-cyclopropylamino}-4-oxo-4H-benzo[d][1,3]oxazin-6-yl)-carbamic acid tert-butyl ester (Compound No.127). Mass spectrum (LCMS, pos: 494 (MH+)). HPLC retention time (A): 4.20 minutes.
(5-Methyl-2- {1-[methyl-(2-pyridin-4-yl-ethyl)-carbamoyl]-cyclopentylamino}-4-oxo-4H-benzo[d][1,3]oxazin-6-yl)-carbamic acid tert-butyl ester (Compound No.128). Mass spectrum (LCMS, pos: 522 (MH+)). HPLC retention time (A): 5.94 minutes.
(5-Methyl-2-{1-[methyl-(2-pyridin-4-yl-ethyl)-carbamoyl]-cyclohexylamino}-4-oxo-4H-benzo[d][1,3]oxazin-6-yl)-carbamic acid tert-butyl ester (Compound No.129). Mass spectrum (LCMS, pos: 536 (MH+)). HPLC retention time (A): 6.07 minutes.

### Example 3

### Production of [5-methyl-2-({[methyl-(2-pyridin-2-yl-ethyl)-carbamoyl]-methyl}-amino)-4-oxo-4H-benzo[d][1,3]oxazin-6-yl]-carbamic acid tert-butyl ester (Compound No. 130)

The reaction was carried out in accordance with the process shown in formula 15. TEA (2.5 ml, 18.0 mmol) and then TMSC1 (1.5 ml, 12.0 mmol) were added to a suspension of a known compound 5 (880 mg, 3.03 mmol) in DCM (46 ml), and the resultant was stirred at room temperature for 1 hour. This reaction solution (0.5 ml) was fractionated in a solution of free amine (0.10 mmol) in DCM (1 ml) that was prepared in the same manner as in Example 2. After the resultant was shaken at room temperature for 15 hours, DCM (1 ml) and a thioisocyanate resin (2.61 mmol/g, 200 mg) were added thereto, and the product was shaken at room temperature for 8 hours. After the filtration, water (1.0 ml) was added thereto, and the product was stirred at room temperature for 15 minutes. The reaction solution was allowed to pass through a column containing diatomaceous earth (Chem Elute, 3.0 ml, manufactured by Varian), and DCM (1.0 ml × 6) was further allowed to pass therethrough. The effluent was recovered and concentrated, DCM (1.0 ml) and P-EDC (50.0 mg) were added thereto, and the product was shaken at room temperature for 3 hours. After the filtration, the reaction solution was allowed to pass through a column containing diatomaceous earth (Chem Elute, 3.0 ml, manufactured by Varian) that had previously been allowed to retain water (1.0 ml), and DCM (1.0 ml × 3) was further allowed to pass therethrough. The effluent was recovered and concentrated. Thus, the title compound of interest was obtained. HPLC retention time (A): 5.22 minutes.

The following compounds were prepared in the same manner, except that other suitable forms of Boc-amino acid I and pyridine ring-containing primary or secondary amine 2 were used.
[5-Methyl-2-(methyl-{[methyl-(2-pyridin-2-yl-ethyl)-carbamoyl]-methyl}-amino)-4-oxo-4H-benzo[d][1,3]oxazin-6-yl]-carbamic acid tert-butyl ester (Compound No.131). HPLC retention time (A): 5.57 minutes.
(5-Methyl-2-{1R-[methyl-(2-pyridin-2-yl-ethyl)-carbamoyl]-ethylamino}-4-oxo-4H-benzo[d][1,3]oxazin-6-yl)-carbamic acid tert-butyl ester (Compound No.132). HPLC retention time (A): 5.59 minutes.
(5-Methyl-2-{2-[methyl-(2-pyridin-2-yl-ethyl)-carbamoyl]-ethylamino}-4-oxo-4H-benzo[d][1,3]oxazin-6-yl)-carbamic acid tert-butyl ester (Compound No.133). HPLC retention time (A): 5.20 minutes.

### Example 4

A process for producing 2-(6-amino-5-methyl-4-oxo-4H-benzo[d][1,3]oxazin-2-ylamino)-2,N-diethyl-N-(2-pyridin-2-yl-ethyl)-propionamide (hereafter referred to as "a Boc-deprotected form of the Compound No.18")

The Compound No.18 (320 mg, 0.65 mmol) was dissolved in 16 ml of AcOEt, and a solution of 4N HCl-AcOEt (16 ml) was added thereto. After being stirred at room temperature for 3 hours, the solvent was removed by distillation under reduced pressure, AcOEt (50 ml) and aqueous NaHCO₃ (saturated aqueous NaHCO₃:water = 1:1, 50 ml) were added to the residue, and the product was stirred until a homogenous two-phase system was obtained. The upper phase was separated, the lower phase was extracted with the aid of AcOEt (50 ml), and these phases were dried over MgSO₄ in combination. After the concentration under reduced pressure, a Boc-deprotected form of the Compound No.18 (239 mg, yield 93.4%) was obtained as a yellow solid. The resultant was stored in an AcOEt solution (0.01M).
1H-NMR (300 MHz, CDCl₃): 1.67 (s, 6H), 2.59 (s, 3H), 3.02 (m, 2H), 3.06 (s, 3H), 3.77 (br, 2H), 7.12 (m, 4H), 7.53 (m, 1H), 7.83 (br, 1H), 8.50 (m, 1H). Mass spectrum (FAB, pos: 396 (MH+)).

### Example 5

### Production of thiophene-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.134)

The reaction was carried out in accordance with the process shown in formula 16. The solvent was removed by distillation from a solution of a Boc-deprotected form of the Compound No.18 in AcOEt to prepare a 0.01M DCM solution. The resultant was transferred to a 2-ml reaction vessel, and TEA (0.012 ml, 0.09 mmol) and thenoyl chloride (0.0066 ml, 0.06 mmol) were added thereto. After the product was shaken at room temperature for 30 minutes, the reaction solution was allowed to pass through a column containing diatomaceous earth (load: 0.564 g) that had previously been allowed to retain water (0.5 ml), and DCM (1.0 ml) was further allowed to pass therethrough. A polyamine resin (77 mg) was added thereto, and the resultant was shaken at room temperature for 15 minutes. After the filtration, a polyamine resin (77 mg) was added, the resultant was shaken at room temperature for 15 minutes, the resultant was filtered, and the solvent was removed by distillation under reduced pressure. Thus, the title compound (6.0 mg, yield 59.4%) was obtained as a light yellow solid. Mass spectrum (FAB, pos): 506 (MH+). HPLC retention time (A): 3.36 minutes.

The following compounds were prepared in the same manner as in Examples 4 and 5, except that other suitable forms of oxazinone derivative and an acylation agent were used instead of the Compound No. 18 and thenoyl chloride.
Thiophene-2-carboxylic acid (5-methyl-2- {1S-[methyl-(2-pyridin-2-yl-ethyl)-carbamoyl]-ethylamino}-4-oxo-4H-benzo[d][1,3]oxazin-6-yl)-amide (Compound No.135). HPLC retention time (A): 3.24 minutes.
2,2-Dimethyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-propionamide (Compound No.136). Mass spectrum (LCMS, pos): 480 (MH+). HPLC retention time (A): 3.97 minutes.
2-[6-(2-Methoxy-acetylamino)-5-methyl-4-oxo-4H-benzo[d][1,3]oxazin-2-ylamino]-2,N-dimethyl-N-(2-pyridin-2-yl-ethyl)-propionamide (Compound No.137). Mass spectrum (LCMS, pos): 467 (MH+). HPLC retention time (A): 1.42 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.138). Mass spectrum (LCMS, pos): 500 (MH+). HPLC retention time (A): 4.48 minutes.
Furan-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.139). Mass spectrum (LCMS, pos): 490 (MH+). HPLC retention time (A): 2.38 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-2-nitro-benzamide (Compound No.140). Mass spectrum (LCMS, pos): 545 (MH+). HPLC retention time (A): 4.04 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-3-nitro-benzamide (Compound No.141). Mass spectrum (LCMS, pos): 545 (MH+). HPLC retention time (A): 5.36 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-4-nitro-benzamide (Compound No.142). Mass spectrum (LCMS, pos): 545 (MH+). HPLC retention time (A): 5.40 minutes.
3-Cyano-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.143). Mass spectrum (LCMS, pos): 525 (MH⁺). HPLC retention time (A): 4.72 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-2-trifluoromethyl-benzamide (Compound No.144). Mass spectrum (LCMS, pos): 568 (MH+). HPLC retention time (A): 5.57 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-3-trifluoromethyl-benzamide (Compound No.145). Mass spectrum (LCMS, pos): 568 (MH⁺). HPLC retention time (A): 6.29 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-4-trifluoromethyl-benzamide (Compound No.146). Mass spectrum (LCMS, pos): 568 (MH+). HPLC retention time (A): 6.28 minutes.
2-Fluoro-N-(5-methyl-2-{1-methyl-1-[methyl-(2-pyridin-2-yl-ethyl)-carbamoyl]-ethylamino) -4-oxo-4H-benzo[d][1,3]oxazin-6-yl)-benzamide (Compound No.147). Mass spectrum (LCMS, pos): 518 (MH+). HPLC retention time (A): 5.08 minutes.
3-Fluoro-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.148). Mass spectrum (LCMS, pos): 518 (MH+). HPLC retention time (A): 5.28 minutes.
4-Fluoro-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.149). Mass spectrum (LCMS, pos): 518 (MH+). HPLC retention time (A): 5.12 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-tetraphthalic acid methyl ester (Compound No.150). Mass spectrum (LCMS, pos): 558 (MH+). HPLC retention time (A): 5.04 minutes.
2-Chloro-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.151). Mass spectrum (LCMS, pos): 534 (MH+). HPLC retention time (A): 5.00 minutes.
3-Chloro-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.152). Mass spectrum (LCMS, pos): 534 (MH⁺). HPLC retention time (A): 5.88 minutes.
4-Chloro-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.153). Mass spectrum (LCMS, pos): 534 (MH+). HPLC retention time (A): 5.85 minutes.
4-Bromo-N-(5-methyl-2-{1-methyl-1-[methyl-(2-pyridin-2-yl-ethyl)-carbamoyl]-ethylamino) -4-oxo-4H-benzo[d][1,3]oxazin-6-yl)-benzamide (Compound No.154). Mass spectrum (LCMS, pos): 578 (MH+). HPLC retention time (A): 6.03 minutes.
2-Iodo-N-(5-methyl-2-{1-methyl-1-[methyl-(2-pyridin-2-yl-ethyl)-carbamoyl]-ethylamino}-4-oxo-4H-benzo[d][ 1,3]oxazin-6-yl)-benzamide (Compound No. 155). Mass spectrum (LCMS, pos): 626 (MH+). HPLC retention time (A): 5.52 minutes.
2-Methyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.156). Mass spectrum (LCMS, pos): 514 (MH⁺). HPLC retention time (A): 5.12 minutes.
3-Methyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No. 157). Mass spectrum (LCMS, pos): 514 (MH⁺). HPLC retention time (A): 5.58 minutes.
4-Methyl-N-(5-methyl-2-{1-methyl-1-[methyl-(2-pyridin-2-yl-ethyl)-carbamoyl]-ethylamino}-4-oxo-4H-benzo[d][ 1,3]oxazin-6-yl)-benzamide (Compound No.158). Mass spectrum (LCMS, pos): 514 (MH+). HPLC retention time (A): 5.54 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-3-phenyl-acrylamide (Compound No.159). Mass spectrum (LCMS, pos): 526 (MH+). HPLC retention time (A): 5.77 minutes.
2-[6-(3-Cyclohexyl-propionylamino)-5-methyl-4-oxo-4H-benzo[d][1,3]oxazin-2-ylamino]-2,N-dimethyl-N-(2-pyridin-2-yl-ethyl)-propionamide (Compound No.160). Mass spectrum (LCMS, pos): 534 (MH⁺). HPLC retention time (A): 6.58 minutes.
2-Methoxy-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.161). Mass spectrum (LCMS, pos): 530 (MH⁺). HPLC retention time (A): 5.70 minutes.
3-Methoxy-N-(5-methyl-2-{1-methyl-1-[methyl-(2-pyridin-2-yl-ethyl)-carbamoyl]-ethylamino}-4-oxo-4H-benzo[d][ 1,3]oxazin-6-yl)-benzamide (Compound No.162). Mass spectrum (LCMS, pos): 530 (MH⁺). HPLC retention time (A): 5.14 minutes.
4-Methoxy-N-(5-methyl-2-{1-methyl-1-[methyl-(2-pyridin-2-yl-ethyl)-carbamoyl]-ethylamino}-4-oxo-4H-benzo[d][ 1,3]oxazin-6-yl)-benzamide (Compound No.163). Mass spectrum (LCMS, pos): 530 (MH+). HPLC retention time (A): 4.90 minutes.
4-Ethoxy-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.164). Mass spectrum (LCMS, pos): 544 (MH+). HPLC retention time (A): 5.66 minutes.
4-Butoxy-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.165). Mass spectrum (LCMS, pos): 572 (MH+). HPLC retention time (A): 6.81 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-4-propyl-benzamide (Compound No.166). Mass spectrum (LCMS, pos): 542 (MH+). HPLC retention time (A): 6.60 minutes.
4-Butyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No. 167). Mass spectrum (LCMS, pos): 556 (MH+). HPLC retention time (A): 7.09 minutes.
4-tert-Butyl-N-(5-methyl-2-{1-methyl-1-[methyl-(2-pyridin-2-yl-ethyl)-carbamoyl]-ethylamino}-4-oxo-4H-benzo[d][ 1,3]oxazin-6-yl)-benzamide (Compound No.168). Mass spectrum (LCMS, pos): 556 (MH+). HPLC retention time (A): 6.82 minutes.
Biphenylyl-4-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.169). Mass spectrum (LCMS, pos): 576 (MH+). HPLC retention time (A): 6.68 minutes.
3-Chloromethyl-N-(5-methyl-2-{1-methyl-1-[methyl-(2-pyridin-2-yl-ethyl)-carbamoyl]-ethylamino}-4-oxo-4H-benzo[d][ 1,3]oxazin-6-yl)-benzamidc (Compound No.170). Mass spectrum (LCMS, pos): 548 (MH⁺). HPLC retention time (A): 5.79 minutes.
4-Chloromethyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.171). Mass spectrum (LCMS, pos): 548 (MH+). HPLC retention time (A): 5.78 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-3-trifluoromethoxy-benzamide (Compound No.172). Mass spectrum (LCMS, pos): 584 (MH+). HPLC retention time (A): 6.42 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-4-trifluoromethoxy-benzamide (Compound No. 173). Mass spectrum (LCMS, pos): 584 (MH+). HPLC retention time (A): 6.42 minutes.
5-tert-Butyl-2-methyl-furan-3-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.174). Mass spectrum (LCMS, pos): 560 (MH+). HPLC retention time (A): 7.00 minutes.
5-Methyl-2-trifluoromethyl-furan-3-carboxylic acid (5-methyl-2-{1-methyl-1-[methyl-(2-pyridin-2-yl-ethyl)-carbamoyl]-ethylamino}-4-oxo-4H-benzo[d][ 1,3]oxazin-6-yl)-amide (Compound No.175). Mass spectrum (LCMS, pos): 572 (MH+). HPLC retention time (A): 6.05 minutes.
2,5-Dimethyl-furan-3-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.176). Mass spectrum (LCMS, pos): 518 (MH+). HPLC retention time (A): 5.55 minutes.
5-Nitro-furan-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.177). Mass spectrum (LCMS, pos): 535 (MH+). HPLC retention time (A): 4.66 minutes.
3-Chloro-benzo[b]thiophene-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.178). Mass spectrum (LCMS, pos): 590 (MH+). HPLC retention time (A): 7.10 minutes.
3-Chloro-thiophene-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.179). Mass spectrum (LCMS, pos): 540 (MH+). HPLC retention time (A): 5.75 minutes.
3-Chloro-4-(propane-2-sulfonyl)-thiophene-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.180). Mass spectrum (LCMS, pos): 646 (MH+). HPLC retention time (A): 5.61 minutes.
2,5-Dichloro-thiophene-3-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.181). Mass spectrum (LCMS, pos): 574 (MH+). HPLC retention time (A): 6.46 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-2-trifluoromethoxy-benzamide (Compound No.182). Mass spectrum (LCMS, pos): 584 (MH+). HPLC retention time (A): 5.88 minutes.
3-Dichloromethyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.183). Mass spectrum (LCMS, pos): 582 (MH+). HPLC retention time (A): 6.31 minutes.
2-[6-(2-Benzyloxy-acetylamino)-5-methyl-4-oxo-4H-benzo[d][1,3]oxazin-2-ylamino]-2,N-dimethyl-N-(2-pyridin-2-yl-ethyl)-propionamide (Compound No.184). Mass spectrum (LCMS, pos): 544 (MH+). HPLC retention time (A): 5.70 minutes.
N-(5-Methyl-2-{1-methyl-1-[methyl-(2-pyridin-2-yl-ethyl)-carbamoyl]-ethylamino}-4-oxo-4H-benzo[d][1,3]oxazin-6-yl)-4-pentyl-benzamide (Compound No.185). Mass spectrum (LCMS, pos): 570 (MH+). HPLC retention time (A): 7.56 minutes.
Benzoic acid 2-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-benzyl ester (Compound No.186). Mass spectrum (LCMS, pos): 634 (MH+). HPLC retention time (A): 6.45 minutes.
4-Ethyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No. 187). Mass spectrum (LCMS, pos): 528 (MH+). HPLC retention time (A): 6.09 minutes.
Cyclopentanecarboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.188). Mass spectrum (LCMS, pos): 492 (MH+). HPLC retention time (A): 4.41 minutes.
2-[6-(2-Chloro-acetylamino)-5-methyl-4-oxo-4H-benzo[d][1,3]oxazin-2-ylamino]-2,N-dimethyl-N-(2-pyridin-2-yl-ethyl)-propionamide (Compound No.189). Mass spectrum (LCMS, pos): 472 (MH+). HPLC retention time (A): 1.97 minutes.
Naphthalene-1-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.190). Mass spectrum (LCMS, pos): 550 (MH+). HPLC retention time (A): 5.96 minutes.
3,5-Dimethoxy-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.191). Mass spectrum (LCMS, pos): 560 (MH+). HPLC retention time (A): 5.49 minutes.
Acetic acid (5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-phenyl-methyl ester (Compound No.192). Mass spectrum (LCMS, pos): 572 (MH+). HPLC retention time (A): 5.12 minutes.
Benzo[1,3]dioxol-5-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No. 193). Mass spectrum (LCMS, pos): 544 (MH+). HPLC retention time (A): 4.59 minutes.
Naphthalene-2-carboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.194). Mass spectrum (LCMS, pos): 550 (MH⁺). HPLC retention time (A): 6.14 minutes.
2-(6-Isobutyrylamino-5-methyl-4-oxo-4H-benzo[d][1,3]oxazin-2-ylamino)-2,N-dimethyl-N-(2-pyridin-2-yl-ethyl)-propionamide (Compound No.195). Mass spectrum (LCMS, pos): 466 (MH+). HPLC retention time (A): 2.02 minutes.
Octanoic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.196). Mass spectrum (LCMS, pos): 522 (MH+). HPLC retention time (A): 6.57 minutes.
2-Ethyl-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-butylamide (Compound No.197). Mass spectrum (LCMS, pos): 494 (MH+). HPLC retention time (A): 4.49 minutes.
Cyclobutanecarboxylic acid (5-methyl-2-{1-methyl-1-[methyl-(2-pyridin-2-yl-ethyl)-carbamoyl]-ethylamino}-4-oxo-4H-benzo[d][1,3]oxazin-6-yl)-amide (Compound No.198). Mass spectrum (LCMS, pos): 478 (MH+). HPLC retention time (A): 2.52 minutes.
2-Bromo-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.199). Mass spectrum (LCMS, pos): 578 (MH+). HPLC retention time (A): 5.24 minutes.
3-Bromo-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-benzamide (Compound No.200). Mass spectrum (LCMS, pos): 578 (MH+). HPLC retention time (A): 6.01 minutes.

### Example 6

### Production of [2-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-ethyl]-carbamic acid tert-butyl ester (Compound No.201)

The reaction was carried out in accordance with the process shown in formula 17. The solvent was removed by distillation from a solution of a Boc-deprotected form of the Compound No.18 in ethyl acetate to prepare a 0.01M DCM solution. Boc-β alanine (75.7 mg, 0.4 mmol) was dissolved in DCM (5.0 ml), P-EDC (333 mg) was added thereto, and the resultant was stirred at room temperature for 30 minutes. The solution of a Boc-deprotected form of the Compound No.18 in 0.01M DCM (10 ml) prepared above was added thereto, and the mixture was stirred at room temperature for 15 hours. After the filtration, the solution was concentrated under reduced pressure, and the resulting crude product was purified by silica gel column chromatography (eluent; CHCl₃:MeOH = 40:1). Thus, the title compound (38.7 mg, yield 68.4%) was obtained as a colorless solid. Mass spectrum (FAB, pos): 567 (MH+). HPLC retention time (A): 2.84 minutes.

The following compounds were prepared in the same manner, except that other suitable forms of oxazinone derivative and acylation agent were used instead of the Compound No.18 and Boc-β alanine.
[2-(5-Methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-ethyl]-carbamic acid benzyl ester (Compound No.202). Mass spectrum (LCMS, pos): 601 (MH+). HPLC retention time (A): 4.21 minutes.
[3-(5-Methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-carbamic acid tert-butyl ester (Compound No.203). Mass spectrum (LCMS, pos): 581 (MH+). HPLC retention time (A): 3.32 minutes.
[3-(5-Methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-carbamic acid benzyl ester (Compound No.204). Mass spectrum (LCMS, pos): 615 (MH+). HPLC retention time (A): 4.75 minutes.
[2-(5-Methyl-2-{1S-[methyl-(2-pyridin-2-yl-ethyl)-carbamoyl]-ethylamino}-4-oxo-4H-benzo[d][1,3]oxazin-6-ylcarbamoyl)-ethyl]-carbamic acid tert-butyl ester (Compound No.205). Mass spectrum (LCMS, pos): 553 (MH+). HPLC retention time (A): 2.78 minutes.
[2-(5-Methyl-2-{1S-[methyl-(2-pyridin-2-yl-ethyl)-carbamoyl]-ethylamino}-4-oxo-4H-benzo[d][1,3]oxazin-6-ylcarbamoyl)-ethyl]-carbamic acid benzyl ester (Compound No.206). HPLC retention time (A): 4.09 minutes.
[3-(5-Methyl-2-{1S-[methyl-(2-pyridin-2-yl-ethyl)-carbamoyl]-ethylamino}-4-oxo-4H-benzo[d][1,3]oxazin-6-ylcarbamoyl)-propyl]-carbamic acid tert-butyl ester (Compound No.207). Mass spectrum (LCMS, pos): 567 (MH+). HPLC retention time (A): 3.44 minutes.
[3-(5-Methyl-2-{1S-[methyl-(2-pyridin-2-yl-ethyl)-carbamoyl]-ethylamino}-4-oxo-4H-benzo[d][1,3]oxazin-6-ylcarbamoyl)-propyl]-carbamic acid benzyl ester (Compound No.208). HPLC retention time (A): 4.69 minutes.

### Example 7

### Production of thiophene-2-carboxylic acid [2-(5-methyl-2-{1-methyl-1-[methyl-(2-pyridin-2-yl-ethyl)-carbamoyl]-ethylamino}-4-oxo-4H-benzo[d][ 1,3]oxazin-6-ylcarbamoyl)-ethyl]-amide (Compound No.209)

The reaction was carried out in accordance with the process shown in formula 17. The Compound No.201 (10.8 mg, 0.02 mmol) was dissolved in 1,4-dioxane (1.0 ml), 4N HCl-1,4-dioxane (1.0 ml) was added thereto, and the mixture was then stirred at room temperature for 1 hour. The solvent was removed by distillation under reduced pressure, DCM (2.0 ml) was added thereto, and TEA (0.017 ml, 0.12 mmol) and thenoyl chloride (0.0064 ml, 0.06 mmol) were then added. After being shaken at room temperature for 30 minutes, the reaction solution was allowed to pass through a column containing diatomaceous earth (load: 0.564 g) that had previously been allowed to retain water (0.5 ml), and DCM (1.0 ml) was further allowed to pass therethrough. A polyamine resin (40 mg) was added, and the product was shaken at room temperature for 15 minutes. After the filtration, a polyamine resin (40 mg) was added, the product was shaken at room temperature for 15 minutes, the resultant was filtered, and the solvent was removed by distillation under reduced pressure. Thus, the title compound (4.9 mg, yield 42.5%) was obtained as a light yellow solid. Mass spectrum (LCMS, pos): 577 (MH+). HPLC retention time (A): 1.54 minutes.

The following compounds were prepared in the same manner, except that other suitable forms of oxazinone derivative and acylation agent were used.
N-[2-(5-Methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-ethyl]-4-trifluoromethyl-benzamide (Compound No.210). HPLC retention time (A): 4.95 minutes.
4-Butyl-N-[2-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-ethyl]-benzamide (Compound No.211). Mass spectrum (LCMS, pos): 627 (MH+). HPLC retention time (A): 6.00 minutes.
Thiophene-2-carboxylic acid [3-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-amide(Compound No.212). HPLC retention time (A): 1.80 minutes.
N-[3-(5-Methyl-2-{1-methyl-1-[methyl-(2-pyridin-2-yl-ethyl)-carbamoyl]-ethylamino}-4-oxo-4H-benzo[d][ 1,3]oxazin-6-ylcarbamoyl)-propyl]-4-trifluoromethyl-benzamide (Compound No.213). Mass spectrum (LCMS, pos): 653 (MH+). HPLC retention time (A): 5.25 minutes.
4-Butyl-N-[3-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-benzamide (Compound No.214). HPLC retention time (A): 6.10 minutes.
Thiophene-2-carboxylic acid [2-(5-methyl-2-{1S-[methyl-(2-pyridin-2-yl-ethyl)-carbamoyl]-ethylamino}-4-oxo-4H-benzo[d][1,3]oxazin-6-ylcarbamoyl)-ethyl]-amide (Compound No.215). HPLC retention time (A): 1.47 minutes.
N-[2-(5-Methyl-2-{1S-[methyl-(2-pyridin-2-yl-ethyl)-carbamoyl]-ethylamino}-4-oxo-4H-benzo[d][ 1,3]oxazin-6-ylcarbamoyl)-ethyl]-4-trifluoromethyl-benzamide (Compound No.216). HPLC retention time (A): 4.86 minutes.
4-Butyl-N-[2-(5-methyl-2-{1S-[methyl-(2-pyridin-2-yl-ethyl)-carbamoyl]-ethylamino}-4-oxo-4H-benzo[d][1,3]oxazin-6-ylcarbamoyl)-ethyl]-benzamide (Compound No.217). HPLC retention time (A): 5.92 minutes.
Thiophene-2-carboxylic acid [3-(5-methyl-2-{1S-[methyl-(2-pyridin-2-yl-ethyl)-carbamoyl]-ethylamino}-4-oxo-4H-benzo[d][1,3]oxazin-6-ylcarbamoyl)-propyl]-amide (Compound No.218). HPLC retention time (A): 1.71 minutes.
N-[3-(5-Methyl-2-{1S-[methyl-(2-pyridin-2-yl-ethyl)-carbamoyl]-ethylamino}-4-oxo-4H-benzo[d][1,3]oxazin-6-ylcarbamoyl)-propyl]-4-trifluoromethyl-benzamide (Compound No.219). HPLC retention time (A): 5.14 minutes.
4-Butyl-N-[3-(5-methyl-2- {1S-[methyl-(2-pyridin-2-yl-ethyl)-carbamoyl]-ethylamino}-4-oxo-4H-benzo[d][1,3]oxazin-6-ylcarbamoyl)-propyl]-benzamide (Compound No.220). HPLC retention time (A): 6.08 minutes.
Pyridine-2-carboxylic acid [3-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-amide (Compound No.221). HPLC retention time (B): 6.46 minutes.
N-[3-(5-Methyl-2- {1-methyl-1-[methyl-(2-pyridin-2-yl-ethyl)-carbamoyl]-ethylamino}-4-oxo-4H-benzo[d][1,3]oxazin-6-ylcarbamoyl)-propyl]-nicotinamide (Compound No.222). HPLC retention time (B): 6.60 minutes.
N-[3-(5-Methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-nicotinamide (Compound No.223). HPLC retention time (B): 7.08 minutes.
4-Dimethylamino-N-[3-(5-methyl-2-{1-methyl-1-[methyl-(2-pyridin-2-yl-ethyl)-carbamoyl]-ethylamino}-4-oxo-4H-benzo[d][1,3]oxazin-6-ylcarbamoyl)-propyl]-benzamide (Compound No.224). HPLC retention time (B): 7.70 minutes.
4-Amino-N-(5-methyl-2-{1-methyl-1-[methyl-(2-pyridin-2-yl-ethyl)-carbamoyl]-ethylamino}-4-oxo-4H-benzo[d][1,3]oxazin-6-yl)-butylamide dihydrochloride (Compound No.225). HPLC retention time (B): 0.69 minutes.

### Example 8

### Production of [2-(5-methyl-2,4-dioxo-1,4-dihydro-2H-benzo[d][1,3]oxazin-6-ylcarbamoyl)-ethyl]-carbamic acid tert-butyl ester (Compound No. 16)

Boc-β alanine (8.9 g, 47.0 mmol) was dissolved in DMF (200 ml), EDC (4.5 g, 23.5 mmol) was added thereto, and the resultant was then stirred at room temperature for 30 minutes. A known compound, 6-amino-5-methyl-1H-benzo[d][1,3]oxazin-2,4-dinone 14 (3.0 g, 15.6 mmol), was added, and the resultant was further stirred at room temperature for 15 hours. Boc-β alanine (1.5 g, 7.8 mmol) and EDC (3.0 g, 15.6 mmol) were further added, the mixture was stirred at room temperature for 2 hours, the product was placed in a reaction vessel containing 800 ml of water, and the resultant was then stirred for 5 minutes. The solid was recovered by filtration, washed with water (150 ml × 3), and then dried under reduced pressure in the presence of phosphorus pentoxide for 72 hours. The dried solid was placed in DCM (150 ml) and then subjected to sonication for 10 minutes. The solid was recovered by filtration, washed with DCM (50 ml x 2), and dried under reduced pressure for 24 hours. Thus, the title compound (4.97 mg, yield 88.0%) was obtained as a colorless solid.
1H-NMR (300 MHz, CDCl₃): 1.38 (s, 9H), 2.46 (s, 3H), 2.50 (m, 2H), 6.98 (d, J = 8.6 Hz, 1H), 7.58 (d, J = 8.6 Hz, 1H), 9.57 (s, 1H), 11.62 (s, 1H). Mass spectrum (FAB, pos: 363 (M+)).

### Example 9

### Production of {2-[5-methyl-2-(methyl-{[methyl-(2-pyridin-2-yl-ethyl)-carbamoyl]-methyl}-amino)-4-oxo-4H-benzo[d][1,3]oxazin-6-ylcarbamoyl]-ethyl}-carbamic acid tert-butyl ester (Compound No.226)

The reaction was carried out in accordance with the process shown in formula 18. TEA (3.8 ml, 27.0 mmol) and then TMSC1 (2.3 ml, 18.0 mmol) were added to a suspension of the Compound No.16 (1.1 g, 3.03 mmol) in DCM (45 ml), and the product was stirred at room temperature for 1 hour. This reaction solution (0.5 ml) was fractionated in the solution of free amine (0.15 mmol) in DCM (1 ml) that was prepared in the same manner as in Example 2. After the product was shaken at room temperature for 15 hours, DCM (1 ml) and a thioisocyanate resin (200 mg) were added, and the product was shaken at room temperature for 8 hours. After the filtration, water (1.0 ml) was added, and the resultant was stirred at room temperature for 15 minutes. The reaction solution was allowed to pass through a column containing diatomaceous earth (Chem Elute, 3.0 ml, manufactured by Varian), and dichloromethane (1.0 ml × 6) was further allowed to pass therethrough. The effluent was collected and concentrated. Thereafter, DCM (1.0 ml) and P-EDC (50.0 mg) were added thereto, and the product was shaken at room temperature for 3 hours. After the filtration, the reaction solution was allowed to pass through a column containing diatomaceous earth (Chem Elute, 3.0 ml, manufactured by Varian) that had previously been allowed to retain water (1.0 ml), and DCM (1.0 ml × 3) was further allowed to pass therethrough. The effluent was collected and concentrated. Thus, the title compound of interest was obtained. HPLC retention time (A): 2.36 minutes.

The following compounds were prepared in the same manner.
[2-(5-Methyl-2-{1R-[methyl-(2-pyridin-2-yl-ethyl)-carbamoyl]-ethylamino}-4-oxo-4H-benzo[d][1,3]oxazin-6-ylcarbamoyl)-ethyl]-carbamic acid tert-butyl ester (Compound No.227). HPLC retention time (A): 2.33 minutes.
{2-[5-Methyl-2-({[methyl-(2-pyridin-2-yl-ethyl)-carbamoyl]-methyl}-amino)-4-oxo-4H-benzo[d][1,3]oxazin-6-ylcarbamoyl]-ethyl}-carbamic acid tert-butyl ester (Compound No.228). HPLC retention time (A): 2.00 minutes.

### Example 10

### Conditions for HPLC assay (A)

Column: Cosmosil 3C18, 4.6 × 50 mm
Mobile phase: 10mM NH₄H₂PO₄(A), acetonitrile (B)
Gradation: A/B = 90/10 (0 minute) → 10/90 (5 minutes) → 10/90 (10 minutes)
Flow rate: 1.0 ml/minute
Wavelength: 254 nm
Shot amount: 5 µl
Assay system:
Pump: Hitachi L-6200
UV detector: Hitachi L-4000
Autosampler: Hitachi AS-2000
Peak analysis: Hitachi D-2500

### Conditions for HPLC assay (B) (illustrative compounds 221 to 225)

Column: Wakosil 2nd HG 3C18, 4.6 × 50 mm
Mobile phase: 10mM NH₄H₂PO₄ (A), acetonitrile (B)
Gradation: A/B = 90/10 (0 minute) → 10/90 (10 minutes) → 10/90 (15 minutes)
Flow rate: 1.0 ml/minute
Wavelength: 254 nm
Shot amount : 5 µl
Assay system:
   Pump: Hitachi L-6200
   UV detector: Hitachi L-4000
   Autosampler: Hitachi AS-2000
   Peak analysis: Hitachi D-2500

### Example 11

### An enzymatic assay method for an HSV-1 protease (assembly) inhibitor

The activity of an assembly protease was determined by fluorescence polarization. Biotin-gamma-amino butyrate-HTYLQASERFRIK-DTAF was used as a fluorescent substrate in accordance with an HSV-1 release and cleavage site. This substrate was incubated with the assembly, and this resulted in the cleavage of the amide bond at the alanine-serine site. Changes in the sizes of fluorescent substrate molecules that increased with the use of avidin as a reaction terminator were assayed by fluorescence polarization.

The test compound was dissolved in DMF, and the resultant was diluted 5-fold with an assay buffer. 6.5 µl thereof was added to wells of a 96-well plate (a U-bottomed 96-well plate, Coster) that had previously been blocked with a blocking solution (10mM Tris-HCl (pH 8.0), 150mM sodium chloride, 0.05% Tween 20, 1 mg/ml of BSA). The enzyme was diluted to 21.3 µg/ml with an assay buffer (1M sodium citrate, 50mM sodium phosphate (pH 7.4), 100mM sodium chloride, 20% glycerol, 2mM TCEP), and 48.5 µl of the resultant was added to each well. The plate was incubated at room temperature for 30 minutes, and 10 µl of 62.5 µg/ml substrate solution was then added to each well. The plate was incubated at room temperature for 60 minutes, and 50 µl of 2 mg/ml avidin phosphate buffer solution (50mM sodium phosphate, 100mM sodium chloride (pH 7.4)) was added to each well. The activity was determined in comparison with a compound without protease inhibitor using a microplate reader for fluorescence polarization. The results are shown in Tables 1 to 6.

### Example 12

### Production of recombinant HSV-1 protease

HSV-1 protease was purified from a vaculovirus that had expressed a DNA construct encoding the reading frame for HSV-1UL26. This construct had further encoded 6 histidine residues at the amino terminus of the protease. These additional histidine residues impart affinity ligands purified by nickel-nitriloacetic acid-agarose (Qiagen). The purified protease was stored as a stock solution for a 20mM HEPES buffer (pH 8.5, containing 20% v/v glycerin). This stock solution was diluted with an assay buffer and then used in the enzyme reaction.

### Example 13

### Production of a substrate

In accordance with the cleavage specificity of the HSV-1 protease at the "release site" of the assembled protein, a specific substrate was synthesized (Diianni et al., J. Biol. Chem. 268, 2048 (1993)). This assembly protein-release site had the sequence ··· HTYLQA*SEKFKMW ···, and the substrate used was biotin-gamma-amino butyrate-HTYLQA*SERFRIK-DTAF. This is a standard method for peptide synthesis, for example, as in the a method described in "The Practice of Peptide Synthesis" (Bodansky and Bodansky (1984)). The product was stored as a 2.5 mg/ml stock solution in DMF. The resultant was diluted to 62.5 µg/ml with a phosphate buffer (50mM sodium phosphate, 100mM sodium chloride, (pH 7.4)) immediately before its use.

### Example 14

### Preparation of an assay buffer

An assay buffer (1M sodium citrate, 50mM sodium phosphate (pH 7.4), 100mM sodium chloride, 20% glycerol, 2mM TCEP) was used to dilute a stock solution for the enzyme and that for the inhibitor.

### Example 15

### An assay method for antiviral activity

These complementary assay methods examine the capacity of a compound to inhibit the generation of new viruses and to inhibit the toxicity of the compound against the host cell. Toxicity indirectly decreases the generation of viruses. Thus, it is important to simultaneously conduct both assays in order to directly compare the results.

Abbreviations:
DMEM; Dulbecco's modified Eagle medium, commercially available
FBS; fetal bovine serum, commercially available, containing unknown factors essential for cell multiplication during culture
HSV; herpes simplex virus

The Vero cells were sown on a 96-well plate and then cultured overnight. The culture supernatant was removed, and 100 µl of 2% FBS-DMEM containing 50,000 plaque-forming units (hereinafter abbreviated as "pfu") of HSV-1 was added. A viral solution was removed and 200 µl of 2% FBS-DMEM comprising a test compound was added. In this case, a substance to which 2% FBS-DMEM not comprising any test compound had been added was determined as a control. These infected cells were incubated in the presence of 5% CO₂ at 37°C for 20 hours, and the medium was once allowed to freeze at -80°C. The culture solution was dissolved, and the amount of viruses in the culture solution was assayed by the plaque method. The concentration of the test compound when it inhibited 90% of plaque formation relative to the control was determined to be EC 90. The results are shown in Tables 1 to 6.

### Example 16

### An assay method for cytotoxicity

The Vero cells were sown on a 96-well plate and then cultured overnight. The culture supernatant was removed, and 200 µl of 2% FBS-DMEM comprising 25 µM of the test compound was added. In this case, a substance to which 2% FBS-DMEM not comprising any test compound had been added was determined as a control. These cells were incubated in the presence of 5% CO₂ at 37°C for 20 hours. Alamar Blue (20 µl) was added to the cells, and cells were incubated for about 6 hours until the color of the control changed. The ratio of the absorbance was assayed using a microplate reader for absorption, and the value relative to the control was determined to be the ratio of living cells. The results are shown in Tables 1 to 6.

### Example 17

### An assay method for human plasma stability

Human plasma was thawed, 200 µl each thereof was placed in each cell of a 96-well plate immediately thereafter, and 4 µl of solution of each test compound in 1 mM DMF was added thereto. A microplate reader for fluorescence absorption was used to assay changes in fluorescence, and half life was determined based on the changes. The results are shown in Table 7.

### Example 18

### An assay method for antiviral activity using mouse models to which ointment for HSV skin infections had been applied

The right abdominal region of a mouse (BALB/c, 5-week-old, female, Charles River) was shaved with clippers, the mouse was subjected to epilation using a depilatory (Divele, Shiseido), and it was then bred for at least 2 days. The entire shaved right abdominal region was coated with the ointment (50 mg) prepared in accordance with Example 19. Two hours thereafter, a roughly 5-mm² portion of the right abdominal region was scratched 15 times with a bundle of ten 25G injection needles, and the mouse was infected with 1 × 10⁵ pfu/3 µl per mouse of the HSV1 7401H strain (distributed from Toyama Medical and Pharmaceutical University). The entire right abdominal region was coated with the ointment 2 hours and 7 hours after the infection. The ointment was applied at 9, 14, and 19 o'clock from the next day to 5 days after the infection. Clinical changes that had occurred up to 10 days after the infection were observed and scored based on the classification as below.
Score 0: not developed
Score 2: white blister
Score 4: orange erosion
Score 6: band erosion
Score 8: red ulcerous band erosion
Score 10: deep-seated lesion
Score 12: death

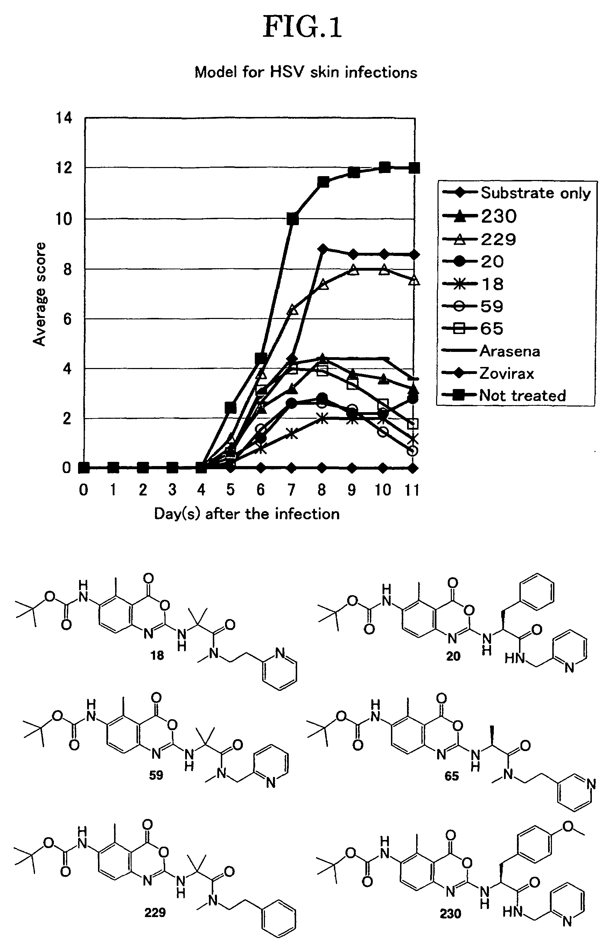

The medicinal effects were evaluated based on the level of the score to be increased. Control groups, a group to which an existing drug, i.e., the Zovirax ointment, was administered, a group to which Arasena-A was administered, a group to which a substrate only was administered, and a group without treatment, were prepared. Illustrative compounds 18, 20, 59, 65, 229, and 230 were used as test compounds. The results are shown in Fig. 1.

### Comparative Example 1

### Production of compounds 229 and 230 (known compounds)

These compounds were produced in the same manner as in Example 1.

{5-Methyl-2-[1-methyl-1-(methyl-phenethyl-carbamoyl)-ethylamino]-4-oxo-4H-benzo[d][1,3]oxazin-6-yl}-carbamic acid tert-butyl ester (compound 229)

1H-NMR (300 MHz, CDCl₃): 1.50 (s, 9H), 1.68 (s, 6H), 2.64 (s, 3H), 2.83 (t, J = 7.2 Hz, 2H), 3.05 (s, 3H), 3.49 (t, J = 7.2 Hz, 2H), 5.62 (br, 1H), 6.23 (s, 1H), 7.23 (m, 6H), 7.81 (br, 1H). HPLC retention time (A): 7.26 minutes

(2-{2-(4-Methoxy-phenyl)-1-[(pyridin-2-ylmethyl)-carbamoyl]-ethylamino}-5-methyl-4-oxo-4H-benzo[d][1,3]oxazin-6-yl)-carbamic acid tert-butyl ester (compound 230)

1H-NMR (300 MHz, CDCl₃): 1.51 (s, 9H), 2.63 (s, 3H), 3.16 (2d, J = 7.4 Hz, 2H), 3.75 (s, 3H), 4.52 (d, J = 5.1 Hz, 2H), 4.68 (m, 1H), 5.49 (br, 1H), 6.27 (s, 1H), 6.77 (d, J = 8.4 Hz, 2H), 7.12 (m, 5sH), 7.59 (td, J = 7.5, 1.8 Hz, 1H), 7.88 (br, 1H), 8.50 (dd, J = 5.1, 1.2 Hz, 1H). HPLC retention time (A): 6.50 minutes.

### Example 19

### Preparation of 5% polyethylene glycol (PEG) ointment

A test compound (500 mg) was added to PEG 400 (4.75 g), and the mixture was dissolved by heating at about 65°C in a water bath. Thereafter, PEG 4000 (4.75 g) was added thereto, and the mixture was dissolved by heating at about 65°C in a water bath. Thereafter, the mixture was thoroughly agitated to solidification. Thus, the ointments of interest were prepared. A control was also prepared in the same manner, except for the use of the test compound.

**Table 1**

| Compound No. | HSV-1 Enzyme inhibition IC50 µM | Antiviral activity HSV-1 EC90 µM | Cytotoxicity Ratio of living cells 25 µM % |
|---|---|---|---|
| 18 | 0.4 | 8.4 | 101.0 |
| 19 | 0.3 | 6.2 | 106.0 |
| 20 | 0.5 | 5.9 | 101.0 |
| 21 | 1.5 | 5.9 | 53.2 |
| 22 | 1.8 | 9.0 | 66.5 |
| 23 | 0.7 | <3.2 | 52.0 |
| 24 | >20 | >25.0 | 99.5 |
| 25 | >20 | >25.0 | 100.0 |
| 26 | 3.2 | 13.0 | 107.0 |
| 27 | 8.7 | >25.0 | 103.0 |
| 28 | 4.4 | <6.3 | 70.0 |
| 29 | 19.5 | 24.3 | 110.0 |
| 30 | 0.7 | 23.8 | 98.6 |
| 31 | >20 | >25.0 | 108.0 |
| 32 | 19.6 | 21.5 | 111.0 |
| 33 | 10.1 | 13.0 | 105.0 |
| 34 | 1.6 | >25.0 | 102.0 |
| 35 | 1.6 | >25.0 | 110.0 |
| 36 | 5.8 | 21.5 | 110.0 |
| 37 | 4.6 | 15.0 | 105.0 |
| 38 | 10.1 | >25.0 | 105.0 |
| 39 | 1.5 | 12.4 | 90.1 |
| 40 | >20 | >25.0 | 100.0 |
| 41 | 5.6 | >25.0 | 101.0 |
| 42 | 1.0 | 10.7 | 83.0 |
| 43 | 0.7 | 9.1 | 104.0 |
| 44 | >20 | 5.9 | 66.2 |
| 45 | 1.7 | 6.6 | 107.0 |
| 46 | 1.7 | 4.9 | 80.1 |
| 47 | 2.9 | <3.2 | 36.7 |
| 48 | 1.2 | 3.7 | 44.2 |
| 49 | 0.9 | <3.2 | 76.0 |
| 50 | 0.9 | <3.2 | 106.0 |
| 51 | 0.6 | 9.3 | 106.0 |
| 52 | 49.0 | 6.0 | 64.5 |

**Table 2**

| Compound No. | HSV-1 Enzyme inhibition IC50 µM | Antiviral activity HSV-1 EC90 µM | Cytotoxicity Ratio of living cells 25 µM % |
|---|---|---|---|
| 53 | 2. 7 | 6.1 | 106. 0 |
| 54 | 1.8 | 6.2 | 62.8 |
| 55 | 7.4 | 5.9 | 43.8 |
| 56 | 3.2 | 3.7 | 77.9 |
| 57 | 1.7 | <3.2 | 71.3 |
| 58 | 1.7 | 10.8 | 105.0 |
| 59 | 1.1 | 10.1 | 106.0 |
| 60 | 26.9 | 4.7 | 89.6 |
| 61 | 3.3 | 4.7 | 107.0 |
| 62 | 2.4 | 4. 7 | 83. 9 |
| 63 | 1.2 | <3.2 | 60.1 |
| 64 | 0.8 | 5.1 | 70. 1 |
| 65 | 0.2 | 7.6 | 104.0 |
| 66 | 14.0 | 4.6 | 81.5 |
| 67 | 1.5 | 5.3 | 96.5 |
| 68 | 2.0 | 6.1 | 66. 9 |
| 69 | 3.6 | <3.2 | 49.5 |
| 70 | 0.9 | <3.2 | 48.0 |
| 71 | 0.8 | >12.5 | 107.0 |
| 72 | 1.1 | >12.5 | 90.7 |
| 73 | 1.0 | >12.5 | 79.8 |
| 74 | 1.5 | >12.5 | 95.3 |
| 75 | 2.2 | >12.5 | 93.1 |
| 76 | 2.1 | >12.5 | 87.9 |
| 77 | 1.8 | >12.5 | 94.4 |
| 78 | 1.4 | >12.5 | 90.7 |
| 79 | 1.0 | 6.0 | 38.5 |
| 80 | 0.9 | 5.9 | 48.0 |
| 81 | 1.7 | 9.5 | 49.7 |
| 82 | 1.5 | 10.8 | 61.6 |
| 83 | 2.6 | 11.5 | 66.6 |
| 84 | 1.7 | 11.0 | 37.6 |
| 85 | 2.3 | 11.7 | 60.7 |
| 86 | 1.1 | >12.5 | 94.6 |
| 87 | 1.7 | >12.5 | 92.4 |

**Table 3**

| Compound No. | HSV-1 Enzyme inhibition IC50 µM | Antiviral activity HSV-1 EC90 µM | Cytotoxicity Ratio of living cells 25 µM % |
|---|---|---|---|
| 88 | 2.7 | >12.5 | 74.1 |
| 89 | 1.4 | >12.5 | 92.6 |
| 90 | 16.9 | >12.5 | 93.6 |
| 91 | >20.0 | >12.5 | 92.3 |
| 92 | >20.0 | >12.5 | 87.2 |
| 93 | >20.0 | >12.5 | 82.4 |
| 94 | 2. 3 | 5. 4 | 55. 2 |
| 95 | 4.4 | 12.3 | 44.0 |
| 96 | 5. | 11.2 | 55.4 |
| 97 | 3.3 | 11.0 | 51. 3 |
| 98 | 6.1 | 5. 8 | 46. 2 |
| 99 | 5.2 | 10. 1 | 53.2 |
| 100 | 9.7 | 11.8 | 44.1 |
| 101 | >20.0 | 8.4 | 45.5 |
| 102 | 1.6 | >12.5 | 64.4 |
| 103 | 2.6 | >12.5 | 68.9 |
| 104 | 4.3 | >12.5 | 63.0 |
| 105 | 2.9 | >12.5 | 55.6 |
| 106 | 7.6 | >12.5 | 53.3 |
| 107 | 7.4 | >12.5 | 45.4 |
| 108 | 4.4 | >12.5 | 97.9 |
| 109 | 1.4 | 8.8 | 58.5 |
| 110 | 6.3 | 7.4 | 71.9 |
| 111 | 3.3 | 7.8 | 80.4 |
| 112 | 2.7 | 10.7 | 84.2 |
| 113 | 10.1 | 8.8 | 71.7 |
| 114 | >10.0 | 5.9 | 76.5 |
| 115 | >20.0 | 6.1 | 71.4 |
| 116 | >10.0 | 6.5 | 68.2 |
| 117 | 0.7 | >12.5 | |
| 118 | 2.3 | >12.5 | |
| 119 | 0.7 | >12.5 | |
| 120 | 1.2 | 10.0 | |
| 121 | 2.8 | 11.5 | |
| 122 | 1. 6 | >12.5 | |

**Table 4**

| Compound No. | HSV-1 Enzyme inhibition IC50 µM | Antiviral activity HSV-1 EC90 µM | Cytotoxicity Ratio of living cells 25 µM % |
|---|---|---|---|
| 123 | 0.6 | 9.6 | |
| 124 | 1.0 | 6.1 | |
| 125 | 2.1 | >12.5 | |
| 126 | 0.4 | 8.6 | |
| 127 | 1.8 | >12.5 | |
| 128 | 0.4 | 10.1 | |
| 129 | 1.1 | 5.8 | |
| 130 | 0.4 | >20.0 | |
| 131 | 0.4 | >20.0 | |
| 132 | 0.8 | 15.8 | |
| 133 | 0.9 | >20.0 | |
| 134 | 0.9 | >12.5 | |
| 135 | 1.3 | . >12.5 | |
| 136 | 4.5 | >12.5 | 81.8 |
| 137 | 4.2 | >12.5 | 98.2 |
| 138 | 2.8 | >12.5 | 84.4 |
| 139 | 1.4 | >12.5 | 90.7 |
| 140 | 4.2 | >12.5 | 96.8 |
| 141 | 8.0 | >12.5 | 80.4 |
| 142 | 1.3 | 10.4 | 61.0 |
| 143 | 3.7 | >12.5 | 86.9 |
| 144 | 2. 3 | >12.5 | 76.1 |
| 145 | 1.3 | 6.0 | 19.7 |
| 146 | 1.2 | 5.6 | 18.0 |
| 147 | 1.6 | 10.2 | 70.2 |
| 148 | 1.5 | 10.3 | 54.6 |
| 149 | 1.5 | 10.6 | 65.4 |
| 150 | 1.4 | 6.1 | 81.5 |
| 151 | 1.4 | 7.6 | 74.2 |
| 152 | 1.8 | 6.3 | 30.9 |
| 153 | 2.6 | 6.2 | 48.6 |
| 154 | 2.4 | 5.4 | 28.3 |
| 155 | 1.5 | >12.5 | 69.1 |
| 156 | 3.8 | >12.5 | 78.0 |
| 157 | 3.3 | 6.2 | 70.5 |

**Table 5**

| Compound No. | HSV-1 Enzyme inhibition IC50 µM | Antiviral activity HSV-1 EC90 µM | Cytotoxicity Ratio of living cells 25 µM % |
|---|---|---|---|
| 158 | 3.3 | 8.3 | 63.6 |
| 159 | 1.4 | 10.8 | 15. 9 |
| 160 | 1.1 | 11.3 | 13.0 |
| 161 | 2.8 | 11.4 | 44.1 |
| 162 | 1.8 | 10.2 | 75.5 |
| 163 | 1.3 | 10.7 | 66.0 |
| 164 | 1.4 | 6.2 | 47.7 |
| 165 | 2.0 | 3.6 | 16.9 |
| 166 | 1.2 | 5.8 | 15.9 |
| 167 | 1.7 7 | 3. 6 | -6.1 |
| 168 | 1.3 | 3.6 | 16.8 |
| 169 | 1.0 | 5.7 | 7.0 |
| 170 | 1.5 | 7.8 | 13.6 |
| 171 | 2.1 | 10.2 | 16.1 |
| 172 | 1.2 | 9.0 | 17.4 |
| 173 | 1.4 | 7.1 | 26.1 |
| 174 | 9.3 | >12.5 | 30.1 |
| 175 | 2.5 | >12.5 | 99.4 |
| 176 | 1.9 | >12.5 | 93.3 |
| 177 | 6.0 | >12.5 | 38.0 |
| 178 | 0.9 | 5. 8 | 28.1 |
| 179 | 1.0 | 6.6 | 61.7 |
| 180 | 1.9 | >12.5 | 87.4 |
| 181 | 1.8 | 10.9 | 44.3 |
| 182 | 4.9 | >12.5 | 95.2 |
| 183 | 1.3 | >12.5 | 101.0 |
| 184 | 2.1 | 12.1 | 93.1 |
| 185 | 7.0 | 3.5 | 29.0 |
| 186 | 1.0 | 3.5 | 18.1 |
| 187 | 1.1 | 4.5 | 65.8 |
| 188 | 1.4 | >12.5 | 91.7 |
| 189 | 2.0 | >12.5 | 41.0 |
| 190 | 1.3 | 12.4 | 74.5 |
| 191 | 0.6 | >12.5 | 89.1 |
| 192 | 7.2 | >12.5 | 93.9 |

**Table 6**

| Compound No. | HSV-1 Enzyme inhibition IC50 µM | Antiviral activity HSV-1 EC90 µM | Cytotoxicity Ratio of living cells 25 µM % |
|---|---|---|---|
| 193 | 3.7 | 12.5 | 67.0 |
| 194 | 6.7 | 11.8 | 71.8 |
| 195 | 2.1 | 12.5 | 99.7 |
| 196 | 0.8 | 11.5 | 86.5 |
| 197 | 7.2 | 12.5 | 82.8 |
| 198 | 3.6 | 12.5 | 99.2 |
| 199 | 3.0 | 4.1 | 56.9 |
| 200 | 1.6 | 3.2 | 21.5 |
| 201 | 1.1 | >12.5 | |
| 202 | 0.5 | >12.5 | |
| 203 | 0.4 | >12.5 | |
| 204 | 0.3 | >12.5 | |
| 205 | 2.5 | >20.0 | |
| 206 | 1.1 | >12.5 | |
| 207 | 1.1 | >12.5 | |
| 208 | 0.2 | >12.5 | |
| 209 | 0.9 | >12.5 | |
| 210 | 0.7 | >12.5 | |
| 211 | 0.7 | >12.5 | |
| 212 | 0.3 | >12.5 | |
| 213 | 0.3 | >12.5 | |
| 214 | 0.3 | >12.5 | |
| 215 | 3.2 | >12.5 | |
| 216 | 3.0 | >12.5 | |
| 217 | 4.7 | >12.5 | |
| 218 | 0.4 | >12.5 | |
| 219 | 0.9 | >12.5 | |
| 220 | 0.5 | >12.5 | |
| 221 | 0.7 | >12.5 | 105.3 |
| 222 | 0.9 | >12.5 | 109.2 |
| 223 | 0.5 | >12.5 | 106.2 |
| 224 | 0.4 | >12.5 | 107.5 |
| 225 | | | |
| 226 | 1.0 | >20.0 | |
| 227 | 2.4 | >20.0 | |
| 228 | 0.7 | >20.0 | |
| 229 | 12.2 | 6.6 | |
| 230 | 0.4 | 6.1 | |

**Table 7**

| Compound No. | Human plasma stability (half-life) minutes |
|---|---|
| 18 | 170 |
| 19 | 372 |
| 20 | 88 |
| 21 | 107 |
| 22 | 181 |
| 23 | 595 |
| 58 | 531 |
| 59 | 308 |
| 65 | 181 |
| 120 | 893 |
| 129 | >900 |
| 230 | 27 |

### Example 20

### An assay method for antiviral activity 2

The HEL cells were sowed on a 48-well plate and then cultured overnight. The culture supernatant was removed, and 100 µl of 2% FBS-DMEM containing 40 plaque-forming units of HSV-1, HSV-2, VZV, or CMV was added. A viral solution was removed 1 hour later, and 500 µl of 2% FBS-DMEM containing 0.5% methylcellulose comprising 50, 25, 12.5, 6.3, 3.2, or 1.6 µM of the test compound was added. In this case, a substance to which 2% FBS-DMEM containing 0.5% methylcellulose not comprising any test compound had been added was designated as a control. In the case of HSV-1, HSV-2, or VZV, incubation was carried out in the presence of 5% CO₂ at 37°C for 3 days. In the case of CMV, incubation was carried out under the same conditions for 14 days. After they were immobilized on formalin and stained with bromophenol blue, the amount of plaque formed was counted, and the ratio of plaque formation of the drug-containing reaction system relative to the control was determined. The concentration of the drug with which the ratio of plaque formation reaches 50% was calculated and determined to be IC50. Concerning HSV-1, an aciclovir-resistant thymidine kinase variant and a DNA polymerase variant were examined in addition to a general aciclovir-sensitive strain. The results are shown in Table 8.

**Table 8**

| Compound No. | HSV1 Sensitive strain | HSV1 Thimidine kinase variant | HSV1 DNA polymerase variant | HSV2 | VZV | HCMV |
|---|---|---|---|---|---|---|
| 18 | 10 | 22.5 | 17.1 | 12.5 | 25 | 11.1 |
| 21 | 6.1 | 16.1 | 6.9 | 10.7 | 6.4 | >5 |
| 22 | 9.5 | 16.3 | 14.3 | 16.6 | >25 | >25 |
| 129 | 5.8 | 17.7 | 8.7 | 10.3 | >5 | >5 |
| Antiviral activity: IC50(µM) | | | | | | |

### Example 21

### Activity of inhibiting virus multiplication through combined use

The Vero cells were sowed on a 48-well plate and then cultured overnight. The culture supernatant was removed, and 100 µl of 2% FBS-DMEM containing 40 plaque-forming units of HSV-1 was added. A viral solution was removed 1 hour later, and 500 µl of 2% FBS-DMEM containing 0.5% methylcellulose comprising the test compound was added. In this case, a substance to which 2% FBS-DMEM containing 0.5% methylcellulose not comprising any test compound had been added was designated as a control. They were incubated in the presence of 5% CO₂ at 37°C for 3 days. After they were immobilized on formalin and stained with bromophenol blue, the amount of plaque formed was counted, and the ratio of plaque formation of the drug-containing reaction system relative to the control was determined. Examination was carried out using the Compound No.18 in combination with aciclovir, the Compound No.18 in combination with penciclovir, and the Compound No.18 in combination with ganciclovir. In this examination, 25, 12.5, 6.3, 3.2, 1.6, or 0 µM of the Compound No.18 was used, and 3.2, 1.6, 0.8, 0.4, 0.2, or 0 µM of aciclovir, penciclovir, or ganciclovir was used.

The Compound No.18 in combination with aciclovir, penciclovir, or ganciclovir synergistically inhibited the multiplication of HSV-1.

### Example 22

### The effect of combined use in mouse models for skin infections

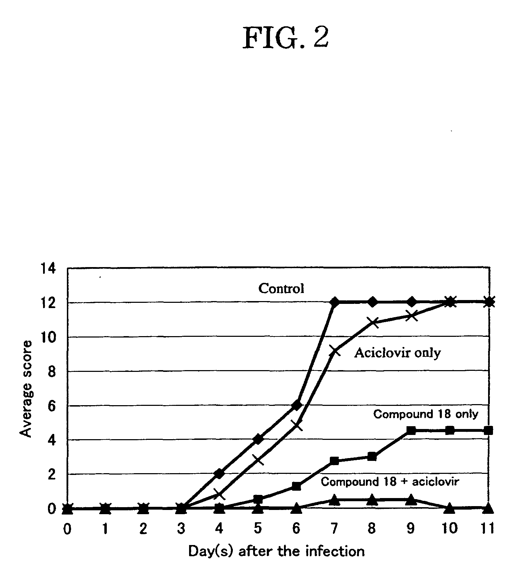

The right abdominal region of a mouse (BALB/c, 5-week-old, female, Charles River) was shaved with clippers, the mouse was subjected to epilation using a depilatory (Divele, Shiseido), and it was bred for at least 2 days. The entire region of the shaved right abdominal region was coated with the ointment (50 mg) of the Compound No.18 prepared in accordance with Example 19. Immediately thereafter, 5 mg/kg of aciclovir was orally administered. Two hours later, a roughly 5-mm² portion of the right abdominal region was scratched 15 times with a bundle of ten 25G injection needles, and the mouse was infected with 1 × 10⁵ pfu/3 pl per mouse of the HSV 1 7401H strain (distributed from Toyama Medical and Pharmaceutical University). The entire right abdominal region was coated with the ointment 2 hours and 7 hours after the infection, and 5 mg/kg of aciclovir was orally administered immediately thereafter. The ointment was applied at 9, 14, and 19 o'clock from the next day to 5 days after the infection, and 5 mg/kg of aciclovir was orally administered immediately thereafter. As controls, a group to which only the ointments of the Compound No.18 was administered and a group to which only acyclovir was administered were simultaneously examined. Clinical changes that had occurred until 11 days after the infection were observed and scored in the same manner as in Example 18. The results are shown in Fig. 2.

All publications, patents, and patent applications cited herein are incorporated herein by reference in their entirety.

### Industrial Applicability

A compound represented by general formula 1 or a pharmaceutically acceptable salt thereof, which is an active ingredient of the present invention, has excellent inhibitory activity against an assembly protease and also has antiviral activity derived therefrom. Also, the use thereof in combination with other antiviral agents results in an enhanced therapeutic effect for herpes infections. Because of its low toxicity, it is useful as a therapeutic agent for herpes virus infections.

## Claims

1. A pyridine ring-containing benzoxazinone derivative or a pharmaceutically acceptable salt or tautomer thereof represented by general formula (1): 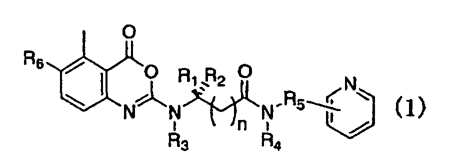 wherein
R₁ and R₂ are independently selected from the group consisting of a hydrogen atom, alkyl, cycloalkyl, cycloalkylalkyl, haloalkyl, guanidinylalkyl, carboxyalkyl, hydroxyalkyl, alkoxyalkyl, aralkoxyalkyl, alkylthioalkyl, alkylsulfinylalkyl, alkylsulfonylalkyl, aryl, heterocyclyl, phenylalkyl, heterocyclylalkyl, and 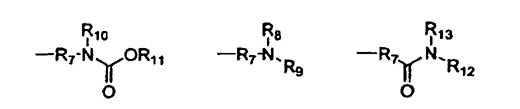 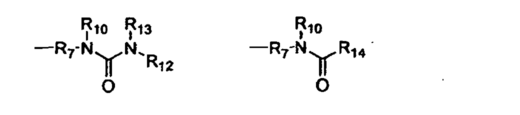 or R₁ and R₂ together form cycloalkyl such as R₁CR₂;
R₃ is selected from the group consisting of a hydrogen atom, alkyl, alkylaminoalkyl, aralkyl, and heterocyclylalkyl or R₁ and R₃ or R₂ and R₃ together form 5- to 7-membered saturated or partially unsaturated heterocyclyl; n is 0 or 1;
R₄ is selected from the group consisting of a hydrogen atom, alkyl, alkenyl, cycloalkyl, cycloalkylalkyl, aryl, heterocyclyl, aralkyl, heterocyclylalkyl, alkylaminoalkyl, and heterocyclylalkyl;
R₅ is alkylene or forms 5- to 7-membered heterocyclyl such as -NR₄R₅ together with R₄;
R₆ is selected from the group consisting of a hydrogen atom, halo, alkyl, and 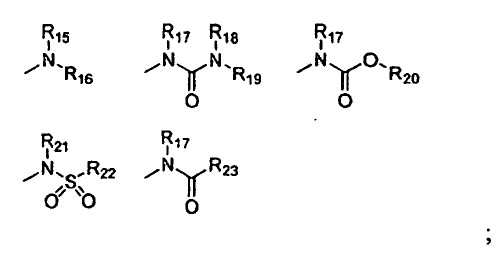 R₇ is alkylene;
R₈, R₉, R₁₂, and R₁₃ are independently selected from the group consisting of a hydrogen atom, alkyl, cycloalkyl, cycloalkylalkyl, aminoalkyl, alkylaminoalkyl, hydroxyalkyl, alkoxyalkyl, aralkoxyalkyl, aryl, heterocyclyl, aralkyl, and heterocyclylalkyl, R₈ and R₉ together form 5- to 7-membered heterocyclyl such as -NR₈R₉, or R₁₂ and R₁₃ together form 5- to 7-membered heterocyclyl such as -NR₁₂R₁₃;
R₁₀ is selected from the group consisting of a hydrogen atom, alkyl, and aralkyl;
R₁₁ is selected from the group consisting of alkyl, cycloalkyl, cycloalkylalkyl, alkylaminoalkyl, aralkoxyalkyl, alkoxyalkyl, aryl, aralkyl, heterocyclyl, and heterocyclylalkyl;
R₁₄ is selected from the group consisting of a hydrogen atom, alkyl, cycloalkyl, cycloalkylalkyl, alkylaminoalkyl, aminoalkyl, carboxyalkyl, aminocarbonylalkyl, hydroxyalkyl, aralkoxyalkyl, alkoxyalkyl, aryl, aralkyl, heterocyclyl, and heterocyclylalkyl;
R₁₅ and R₁₆ are independently selected from the group consisting of a hydrogen atom, alkyl, cycloalkyl, cycloalkylalkyl, aryl, aralkyl, heterocyclyl, heterocyclylalkyl, alkylaminoalkyl, aralkylaminoalkyl, alkoxyalkyl, and aralkoxyalkyl or R₁₅ and R₁₆ together form 5- to 7-membered heterocyclyl such as -NR₁₅R₁₆;
R₁₇ and R₂₁ are independently selected from the group consisting of a hydrogen atom, alkyl, and aralkyl;
R₁₈ and R₁₉ are independently selected from the group consisting of a hydrogen atom, alkyl, cycloalkyl, cycloalkylalkyl, haloalkyl, aminoalkyl, alkylaminoalkyl, carboxyalkyl, aminocarbonylalkyl, alkylaminocarbonylalkyl, hydroxyalkyl, alkoxyalkyl, aralkoxyalkyl, alkylsulfonylalkyl, alkylsulfinylalkyl, alkylthioalkyl, aryl, aralkyl, aralkenyl, heterocyclyl, and heterocyclylalkyl or R₁₈ and R₁₉ together form 5- to 7-membered heterocyclyl such as -NR₁₈R₁₉;
R₂₀ is selected from the group consisting of alkyl, haloalkyl, alkylaminoalkyl, carboxyalkyl, aminocarbonylalkyl, alkylaminocarbonylalkyl, aralkoxyalkyl, alkoxyalkyl, cycloalkyl, cycloalkylalkyl, alkylsulfonylalkyl, alkylsulfinylalkyl, alkylthioalkyl, aryl, aralkyl, heterocyclyl, and heterocyclylalkyl;
R₂₂ is selected from the group consisting of amino, alkyl, alkylamino, alkylaminoalkyl, and aryl;
R₂₃ is selected from the group consisting of a hydrogen atom, alkyl, cycloalkyl, cycloalkylalkyl, haloalkyl, aminoalkyl, alkylaminoalkyl, carboxyalkyl, aminocarbonylalkyl, alkylaminocarbonylalkyl, hydroxyalkyl, alkoxyalkyl, aralkoxyalkyl, alkylsulfonylalkyl, alkylsulfinylalkyl, alkylthioalkyl, aryl, aralkyl, aralkenyl, heterocyclyl, heterocyclylalkyl, and 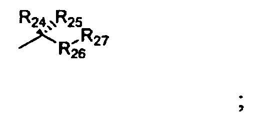 R₂₄ and R₂₅ are independently selected from the group consisting of a hydrogen atom, alkyl, cycloalkyl, cycloalkylalkyl, haloalkyl, guanidinylalkyl, carboxyalkyl, hydroxyalkyl, alkoxyalkyl, aralkoxyalkyl, alkylthioalkyl, alkylsulfinylalkyl, alkylsulfonylalkyl, aryl, heterocyclyl, aralkyl, heterocyclylalkyl, and 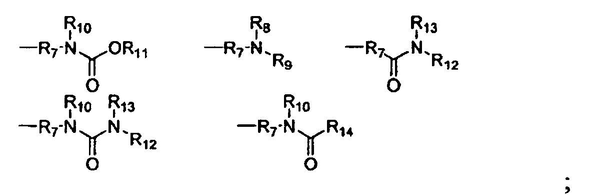 R₂₆ and R₂₈ are independently alkylene; and
R₂₇ is selected from the group consisting of a hydrogen atom, halo, alkyl, and

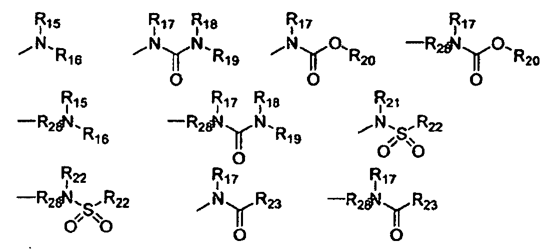

2. The compound according to claim 1, wherein R₁ and R₂ are independently selected from the group consisting of a hydrogen atom, lower alkyl, phenylalkyl, cyclohexylmethyl, and heterocyclylmethyl or R₁ and R₂ together form lower cycloalkyl such as R₁CR₂; R₃ is selected from the group consisting of a hydrogen atom and lower alkyl, n is 1; R₄ is selected from the group consisting of a hydrogen atom and lower alkyl; and R₅ is lower alkylene or forms 5- or 6-membered heterocyclyl together with R₄.

3. The compound according to claim 2, wherein R₁ and R₂ are independently selected from the group consisting of a hydrogen atom, methyl, benzyl, and phenylethyl or R₁ and R₂ together form cyclopropyl, cyclopentyl, or cyclohexyl.

4. The compound according to claim 3, which is selected from the following group of compounds:
(5-methyl-2-{1-methyl-1-[methyl-(2-pyridin-2-yl-ethyl)-carbamoyl]-ethylamino}-4-oxo-4H-benzo[d][1,3]oxazin-6-yl)-carbamic acid tert-butyl ester;
(5-methyl-2-{1-methyl-1-[methyl-(2-pyridin-3-yl-ethyl)carbamoyl]-ethylamino}-4-oxo-4H-benzo[d][1,3]oxazin-6-yl)-carbamic acid tert-butyl ester;
(5-methyl-4-oxo-2- {2-phenyl-1S-[(pyridin-2-ylmethyl)-carbamoyl]-ethylamino}-4H-benzo[d][1,3]oxazin-6-yl)-carbamic acid tert-butyl ester;
(5-methyl-2-{1-[methyl-1S-(2-pyridin-2-yl-ethyl)-carbamoyl]-2-phenyl-ethylamino}-4-oxo-4H-benzo[d][1,3]oxazin-6-yl)-carbamic acid tert-butyl ester;
[5-methyl-2-(methyl-{2-phenyl-1S-[(pyridin-2-ylmethyl)-carbamoyl]-ethyl}-amino)-4-oxo-4H-benzo[d][1,3]oxazin-6-yl]-carbamic acid tert-butyl ester;
{5-methyl-2-[1S-(methyl-pyridin-2-ylmethyl-carbamoyl)-3-phenyl-propylamino]-4-oxo-4H-benzo[d][1,3]oxazin-6-yl)-carbamic acid tert-butyl ester;
{5-methyl-2-[1S-(methyl-pyridin-2-ylmethyl-carbamoyl)-ethylamino]-4-oxo-4H-benzo[d][1,3]oxazin-6-yl)-carbamic acid tert-butyl ester;
{5-methyl-2-[1-methyl-1-(methyl-pyridin-2-ylmethyl-carbamoyl)-ethylamino]-4-oxo-4H-benzo[d][1,3]oxazin-6-yl)-carbamic acid tert-butyl ester;
(5-methyl-2-{1S-[methyl-(2-pyridin-3-yl-ethyl)-carbamoyl]-ethylamino}-4-oxo-4H-benzo[d][1,3]oxazin-6-yl)-carbamic acid tert-butyl ester;
{5-methyl-2-[1-(methyl-pyridin-3-ylmethyl-carbamoyl)-cyclohexylamino]-4-oxo-4H-benzo[d][1,3]oxazin-6-yl}-carbamic acid tert-butyl ester; and
(5-methyl-2-{1-[methyl-(2-pyridin-4-yl-ethyl)-carbamoyl]-cyclohexylamino}-4-oxo-4H-benzo[d][1,3]oxazin-6-yl)-carbamic acid tert-butyl ester.

5. Use of the compound according to any of claims 1 to 4 as medicine.

6. A pharmaceutical composition comprising a therapeutically effective amount of the compound according to any of claims 1 to 4 and a pharmaceutically acceptable carrier or diluent.

7. The pharmaceutical composition according to claim 6 for treating virus infections.

8. The pharmaceutical composition according to claim 7, wherein virus infections are induced by herpes viruses.

9. The pharmaceutical composition according to claim 8, which exhibits an efficacy of treating herpes virus infections by inhibiting protease.

10. The pharmaceutical composition according to claim 8 or 9, wherein virus infections are induced by HSV-1 or HSV-2.

11. The pharmaceutical composition according to claim 8 or 9, wherein virus infections are induced by VZV.

12. The pharmaceutical composition according to claim 8 or 9, wherein virus infections are induced by HCMV.

13. An agent for combination therapy, which simultaneously or sequentially uses an agent containing at least one compound according to claims 1 to 4 and an agent containing a substance having antiviral activities that is different from the former agent.

14. The agent for combination therapy according to claim 13, wherein the antiviral activity is antiherpes virus activity.

15. The agent for combination therapy according to claim 14, wherein the antiherpes virus activity inhibits the DNA synthesis of herpes viruses.

16. The agent for combination therapy according to claim 13, wherein the substance having antiviral activities is aciclovir, penciclovir, valaciclovir, famciclovir, ganciclovir, valganciclovir, foscarnet, cidofovir, vidaravine, epavir, epervudine, idoxuridine, brivudine, viracol, viraplex, histamine dihydrochloride, vaccine preparations, HSV treatment (SLA Pharma), ME-609 or MIV-609 (Medivir), NVTL-001 (Novactyl), AG-701 (Antigenics), HSV inhibitors (Bayer), A-5021 (Ajinomoto Co., Inc.), AV-100 (Ajinomoto Co., Inc.), Pharmaprojects No. 6595 (GlaxoSmithKline), antisense preparations, T-611 (Tularik), CMV cell therapy (Targeted Genetics), fomivirsen sodium, Pharmaprojects No. 6645 (Pharmacia), mizoribine, ribavirin, interferon preparations, resiquimod, or docosonol.

17. The agent for combination therapy according to claim 13, wherein the substance having antiviral activities is aciclovir, penciclovir, valaciclovir, famciclovir, ganciclovir, valganciclovir, foscarnet, cidofovir, vidaravine, or Pharmaprojects No. 6645 (Pharmacia).

18. The agent for combination therapy according to claim 13, wherein the substance having antiviral activities is aciclovir, penciclovir, valaciclovir, famciclovir, ganciclovir, or valganciclovir.
